# EUROPÄISCHE PATENTSCHRIFT

(11) **EP 2 448 893 B1**
(45) Veröffentlichungstag und Bekanntmachung des Hinweises auf die Patenterteilung: **12.08.2015**
(21) Anmeldenummer: 10725455.9
(22) Anmeldetag: 16.06.2010
(51) Int. Cl.: C07C 1/20, C07C 11/09

(54) **HERSTELLUNG VON ISOBUTEN DURCH SPALTUNG VON MTBE**
PREPARATION OF ISOBUTENE BY CLEAVAGE OF MTBE
PREPARATION DU ISOBUTENE PAR CLIVAGE DU MTBE

(30) Priorität: 01.07.2009 DE 102009027404
(43) Veröffentlichungstag der Anmeldung: 09.05.2012
(73) Patentinhaber: Evonik Degussa GmbH, 45128 Essen (DE)
(72) Erfinder: WINTERBERG, Markus, 45711 Datteln (DE); RÖTTGER, Dirk, 50672 Köln (DE); RIX, Armin, 45770 Marl (DE); BUKOHL, Reiner, 45770 Marl (DE); LUH, Walter, 45770 Marl (DE); WIEDERHOLD, Holger, 64295 Darmstadt (DE); SCHILLING, Gunnar, 45701 Herten (DE); BÖING, Christian, 50679 Köln (DE)
(86) Internationale Anmeldenummer: PCT/EP2010/058424
(87) Internationale Veröffentlichungsnummer: WO 2011/000696

(56) Entgegenhaltungen:
- WO-A1-2004/018393
- FR-A1- 2 424 502
- US-A- 4 232 177
- US-A- 4 570 026

## Beschreibung

Die vorliegende Erfindung betrifft ein Verfahren zur Herstellung von Isobuten durch Spaltung von MTBE.

Isobuten ist Ausgangsstoff für die Herstellung einer Vielzahl von Produkten, z. B. für die Herstellung von Butylkautschuk, Polyisobutylen, Isobuten-Oligomeren und t-Butylaromaten. Darüber hinaus kann Isobuten als Vorstufe für die Herstellung von Methacrylsäure und deren Ester verwendet werden.

In technischen Strömen, beispielsweise im C4-Schnitt aus Erdölcrackern, liegt Isobuten häufig zusammen mit gesättigten und ungesättigten C₄-Kohlenwasserstoffen vor. Aus diesen Gemischen kann Isobuten wegen der geringen Siedepunktsdifferenz bzw. des sehr geringen Trennfaktors zwischen Isobuten und 1-Buten durch Destillation nicht wirtschaftlich abgetrennt werden. Daher wird Isobuten aus technischen Kohlenwasserstoffgemischen üblicherweise dadurch gewonnen, dass Isobuten zu einem Derivat umgesetzt wird, das sich leicht vom übrig gebliebenen Kohlenwasserstoffgemisch abtrennen lässt, und dass das isolierte Derivat zu Isobuten und Derivatisierungsmittel zurück gespalten wird.

Aus C₄-Schnitten von Steamcrackern wird Isobuten daher normalerweise wie folgt abgetrennt: Nach Entfernung des größten Teils der mehrfach ungesättigten Kohlenwasserstoffe, hauptsächlich Butadien, durch Extraktion(-sdestillation) oder Selektivhydrierung zu linearen Butenen wird das verbleibende Gemisch (Raffinat I oder hydriertes Crack-C₄) mit Alkohol oder Wasser umgesetzt.
Bei der Umsetzung des Isobutens mit Wasser wird tert.-Butanol (TBA) gebildet, bei der Verwendung von Alkohol entsteht der Alkyl-tert-butylether, wobei die Alkylgruppe durch den verwendeten Alkohol bestimmt ist. Bei Einsatz von Methanol wird Methyl-tert.-butylether (MTBE), der bei Verwendung von Ethanol Ethyl-tert.-butylether (ETBE) gebildet. Beide Komponenten finden primär Einsatz als Komponente zur Erhöhung der Oktanzahl von Otto-Kraftstoffen. Dabei ist die Umsetzung des Isobutens mit Ethanol zu ETBE großtechnisch erst im letzten Jahrzehnt relevant geworden, da die Gesetzgebung einen Anteil an regenerativen Rohstoff im Kraftstoff vorschreibt und Bioethanol in ausreichendem Maße verfügbar ist. Die Veretherung mit C3- oder C4-Alkoholen ist im Rahmen von Verfahren zur Isobutenherstellung ebenfalls beschrieben worden (US 4,287,379; US 4,320,232).
Nach ihrer Abtrennung können sowohl die Ether als auch TBA in Umkehrung ihrer Bildung zu Isobuten gespalten werden.
Der große Vorteil der Umsetzung mit Alkoholen ist, dass hierbei ein sehr hoher Umsatz des Isobutens erreicht werden kann. Dies ist insbesondere notwendig, wenn aus den restlichen C4-Kohlenwasserstoffen, technisch oft als Raffinat II bezeichnet, 1-Buten in einer Polymer-Grade Qualität gewonnen werden soll (Revue De Institut Francais du Petrole, Vol. 46, No. 3, Mai-Juni 1991, Seiten 361 - 387). Der hierfür notwendige Umsatz von typischer Weise > 99,5 % kann bei der Umsetzung mit Methanol in ein- oder mehrstufigen Umsetzungen erreicht werden (Catalysis Today, 1997, Vol. 34, Seiten 447-455). Daneben hat Methanol den Vorteil, dass aus ihm durch Abspaltung von Wasser kein Olefin gebildet werden kann.

Verfahren zur Herstellung von MTBE wurden verstärkt seit den 70er Jahren entwickelt, da MTBE im großen Maße als Kraftstoffzusatz zur Anhebung der Oktanzahl eingesetzt wurde. Ein entscheidender Schritt in der Entwicklung in den folgenden Jahrzehnten war der Einsatz von Reaktivdestillationsverfahren. Diese Technik kommt seit den 90er Jahren großtechnisch zum Einsatz. Trotz des sehr hohen Umsatzes des Isobutens war es mit dieser Technik möglich, den spezifischen Energieeinsatz zu senken. Seitdem hat sich die MTBE-Synthese zu einer der Standardanwendungen für die Reaktivdestillationsverfahren entwickelt (z. B. EP 1 199 296).
Verfahren zur Rückspaltung der Ether sind ebenfalls schon lange bekannt (DE 1 216 865). Großtechnische Anwendung findet die Spaltung von MTBE aber erst seit den 80er Jahren und, insbesondere im Vergleich zur Synthese, nur in sehr begrenztem Umfang.

Die Spaltung des MTBEs kann dabei in der Flüssig- oder Gasphase erfolgen. Typische Nebenreaktionen sind die Bildung von Wasser und Dimethylether (DME) aus Methanol und die Bildung von Di- und Oligomeren des Isobutens (hauptsächlich C8-, C12-Kohlenwasserstoffe).

Die für die MTBE-Synthese eingesetzten C4-Kohlenwasserstoffe sind in der Regel wassergesättigt (ca. 200 - 400 ppm Wasser). Zusätzlich kann Wasser über den eingesetzten Alkohol in die Synthese kommen. Insbesondere die in der MTBE-Spaltung abgetrennten Methanolfraktionen enthalten oft noch Wasser aus der DME-Bildung der Spaltung.

Wasser wird in der MTBE-Synthese zumindest teilweise zu TBA umgesetzt. Der gebildete TBA kann bei der Aufreinigung des Austrags aus der Synthese vor der Spaltung abgetrennt werden. Dadurch geht dieser Anteil des im Rohstoff enthaltenen Isobutens für die Isobutenherstellung verloren. Ziel des erfindungsgemäßen Verfahrens war es daher, den in der Synthese entstandenen TBA zumindest teilweise für die Isobutenherstellung zu nutzen. Auch ist bekannt, dass der Eintrag von Wasser in die Ethersynthese an saueren lonentauschern zu einer deutlichen Verringerung der Reaktionsgeschwindigkeit (Ind. Eng. Chem. Res 1993, 32, 564-569) führt. Eine ausreichende Reaktionsgeschwindigkeit in der MTBE-Synthese ist aber insbesondere dann notwendig, wenn der Umsatz der Ethersynthese ausreichend hoch sein muss, um eine nachgelagerte 1-Buten-Produktion sicher zu stellen.

Die Problematik, dass sich bei der Kopplung von einer MTBE-Synthese mit einer Spaltung, aus der der entstandene Alkohol in die Synthese zurückgeführt wird, Komponenten im Kreislauf anreichern können, ist bekannt. Obwohl diese Kopplung von Synthese und Spaltung Gegenstand diverser Patente ist, finden sich kaum Angaben zu Abtrennung dieser Komponenten, insbesondere nicht zu TBA.

In Hydrocarbon Processing, August 1981, Seiten 101 - 106, geben die Autoren an, dass eine Rückführung des Methanols aus der Spaltung in die Synthese möglich ist, solange eine Restmenge von mindestens 30% MTBE zur Ausschleusung von Nebenkomponenten nicht in der Spaltung verwertet werden. Soll die gesamte MTBE-Menge gespalten werden, wird eine zusätzliche "Recycle purification unit" notwendig. Dabei wird nicht spezifiziert, welche Komponenten und auf welche Art und Weise diese ausgeschleust werden.

In US 4,570,026 wird ebenfalls ein Verfahren mit gekoppelter MTBE-Synthese und Spaltung beschrieben. Im rückgeführten Methanol sind noch Restmengen an MTBE und Oligomere enthalten. Die Oligomere werden über einen MTBE Strom aus der Synthese, der nicht der Spaltung zugeführt wird, ausgeschleust.

US 5,567,860 beschreibt die Abtrennung Trennung von sekundären Ethern aus den Spaltprodukten, wobei die Rückführung des erhaltenen gereinigten Recyclestroms direkt in die Spaltung erfolgt.

Die Patentschrift EP 0 869 107 legt ein Verfahren zur Herstellung von 1-Buten, Isobuten und MTBE offen. Aus einer 1-Buten und Isobuten enthaltenden Mischung von Kohlenwasserstoffen wird das Isobuten durch MTBE-Bildung und Destillation abgetrennt und anschließend katalytisch gespalten. Die Produkte der Spaltung werden in eine Isobuten-reiche Fraktion und eine Fraktion getrennt, die den größten Teil des gebildeten Methanols, gegebenenfalls nicht zersetzten Ether und gegebenenfalls schwere Verbindungen enthält. Diese Fraktion wird gegebenenfalls teilweise in die Synthese zurückgeführt. In speziellen Ausführungsformen wird ein Teil des MTBE als Motortreibstofffraktion genutzt. Bei der im Beispiel angegebenen Aufreinigung der Produkte aus der Spaltungsreaktion wird Wasser mit dem gebildeten Isobuten, das noch Anteile an Methanol enthält, abgetrennt. Die Hauptmenge Methanol, die gegebenenfalls in die Synthese zurückgeführt wird, enthält noch Anteile an nicht umgesetzten MTBE und Dimere des Isobutens. Eine weitere Aufreinigung des Stroms wird nicht beschrieben.

Einige Verfahren setzen bei der Spaltung des Ethers Wasserdampf zu. So beschreibt DE 1 934 422 ein Verfahren zur Abtrennung von tertiären Monoolefinen aus Kohlenwasserstoffgemischen. Nach dem Reaktor wird das Produktgemisch in einem Dekanter in eine wässrige- und eine Organische Phase getrennt. Aus der wässrigen Phase werden Restmengen an enthaltenem Alkohol destillativ abgetrennt und die Synthese zurückgeführt. Das Wasser wird erneut in der Spaltung eingesetzt. Bei der Verfahrensvariante ohne Zusatz von Dampf in der Spaltung erfolgt die Rückführung einer Fraktion in die Synthese, die aus nicht umgesetzten Ethern, Alkohol und in der Regel Wasserspuren besteht.

Die Synthese und Spaltung von MTBE sind auch Gegenstand der Publikation in Catalysis Today, 1997, Vol. 34, Seiten 447-455. Danach ist insbesondere eine zu starke Aufkonzentration vom 2-Methoxybutan (Methyl-sec.-butylether, MSBE) im Methanolkreislauf zu vermeiden. Daneben finden sich Angaben zur Bildung von TBA in der MTBE-Synthese und zur Rückspaltung von TBA unter den Bedingungen der MTBE-Spaltung. Angaben zur Rückführung oder Ausschleusung von TBA oder eine Begrenzung der Wasserkonzentration werden nicht gemacht.

Es bestand daher die Aufgabe, einen Prozess zur Synthese und Spaltung von MTBE zu entwickeln, der das in der MTBE-Synthese durch TBA Bildung gebundene Isobuten für die Spaltung zugänglich macht und gleichzeitig eine übermäßige Anreicherung von TBA und/oder Wasser in Synthese und Spaltung begrenzt.

Diese Aufgabe wurde gelöst durch ein Verfahren zur Herstellung von Isobuten durch Spaltung von MTBE, wobei folgende Schritte durchlaufen werden:
a) MTBE-Synthese; Umsetzung von Isobuten-haltigen Kohlenwasserstoffgemischen (II), mit Methanol (III), enthalten in einem oder mehreren Methanol-haltigen Strömen (VIII, IX), an sauren lonentauschern unter Erhalt eines Stroms (IV), enthaltend MTBE und TBA,
b) MTBE-Abtrennung; destillative Abtrennung eines Stroms (V), enthaltend MTBE und TBA, aus Strom (IV),
c) MTBE-Spaltung; Spaltung des Stroms (V) an einem heterogenen Katalysator in der Gasphase unter Erhalt eines Stroms (VI), enthaltend mindestens Isobuten, Methanol, MTBE und Wasser und gegebenenfalls TBA,
d) Isobuten-Abtrennung; destillative Trennung des Stroms (VI) unter Erhalt eines Stroms (VII), enthaltend jeweils mehr als 50 Massen-% der in Strom (VI) enthaltenen Methanol-, TBA- und Wasser-Mengen, und eines Stroms (XVII), enthaltend Isobuten,
e) Wasser-Abtrennung; destillative Abtrennung von Wasser aus dem Strom (VII) auf unter 1 Massen-% Anteil unter Erhalt eines Stroms (VIII),
f) Rückführung; vollständige oder teilweise Rückführung des methanolhaltigen Stroms (VIII) in die MTBE-Synthese.

Das erfindungsgemäße Verfahren zeichnet sich dadurch aus, dass der Wassergehalt in Verfahrensschritt e) auf unter 1 Massen-% Anteil in der Fraktion (VIII) abgesenkt wird.

Der Vorteil des erfindungsgemäßen Verfahrens liegt nicht nur in der Nutzung des als TBA gebundenen Isobutens für die Spaltung. Als zusätzlicher Strom fällt nur eine relativ geringe Abwassermenge an, die beispielsweise einer Kläranlage zugeführt werden kann. Würde hingegen TBA aus dem Prozess als separater Strom ausgeschleust, müsste dieser getrennt verwertet werden. Der in der Literatur oft angegebene Weg einer Abtrennung bzw. Abmischung in einen Reststrom MTBE, der beispielsweise als Vergaserkraftstoff vermarktet wird, ist aber nur begrenzt möglich. In Europa liegt beispielsweise die typische Spezifikation für marktgängiges MTBE bei 98 % Ethergehalt.

Je nach eingesetztem Katalysator im Verfahrensschritt der Spaltung des Ethers besteht die Gefahr, dass die nachfolgenden Aufarbeitungsschritte einen sauren pH-Wert aufweisen. Ursache kann beispielsweise die Abgabe von Säurespuren durch den Katalysator oder die Bildung von beispielsweise Ameisensäure als Nebenreaktion der Spaltungsreaktion sein. Dies kann zur Folge haben, dass in diesen nachfolgenden Verfahrensschritten keine preiswerten unlegierten Stähle eingesetzt werden können, sondern zur Vermeidung von Korrosion aufgrund der sauren Bedingungen höherwertige, hochlegierte Stähle eingesetzt werden müssen. Um dies zu vermeiden, kann in Verfahrensschritt d) oder in Verfahrensschritt e) des erfindungsgemäßen Verfahrens ein Laugestrom zudosiert werden. Ein weiterer Vorteil des erfindungsgemäßen Verfahrens ist, dass die Lauge auf einfache Weise bei der Wasserabtrennung in Verfahrensschritt e) gemeinsam mit der Wasserfraktion aus dem Verfahren ausgeschleust werden kann. Damit besteht keine Gefahr, dass die die Lauge in Verfahrensschritt a) zurückgeführt wird und hier gegebenenfalls zu einer Schädigung des eingesetzten Katalysators führt.

### Einsatzstoffe

In dem erfindungsgemäßen Verfahren können alle üblicherweise zur Verfügung stehenden technischen C4-Kohlenwasserstoffgemische eingesetzt werden. Geeignete Isobuten-haltige C4-Ströme sind beispielsweise Leichtbenzinfraktionen aus Raffinerien, C4-Fraktionen aus Crackern (beispielsweise Steamcracker, Hydrocracker, Katcracker), Gemische aus Fischer-Tropsch-Synthesen, Gemische aus der Dehydrierung von Butanen, Gemische aus Skelettisomerisierung linearer Butene und Gemische, entstanden durch Metathese von Olefinen. Diese Techniken sind in der Fachliteratur beschrieben (K. Weissermel, H. J. Arpe, Industrielle Organische Chemie, Wiley-VCH, 5. Auflage, 1998, Seiten 23 bis 24; 65 bis 99; 122 bis 124).

Eingesetzt werden beispielsweise C4-Fraktionen aus Steamcrackern, die primär zur Produktion von Ethen und Propen betrieben werden und in denen als Rohstoffe beispielsweise Raffineriegase, Naphtha, Gasöl, LPG (liquified petroleum gas) und NLG (natural liquified gas) eingesetzt werden, oder C4-Fraktionen aus Katcrackern. Die als Nebenprodukt anfallenden C4-Schnitte enthalten je nach Crack-Verfahren unterschiedliche Mengen an Isobuten, 1,3-Butadien, 1-Buten, c-2-Buten, t-2-Buten, n-Butan und i-Butan.

Die im erfindungsgemäßen Verfahren eingesetzten Isobuten-haltigen C4-Kohlenwasserstoffe weisen vorzugsweise einen Gehalt an 1,3-Butadien von kleiner 1 Massen-%, besonders bevorzugt kleiner 0,5 Massen-%, auf. Typische Isobuten-haltige C4-Kohlenwasserstoffgemische sind z.B. hydriertes CC4 (HCC4) und Raffinat I. Hydriertes CC4 wird beispielsweise nach bekannten technischen Verfahren durch selektive Hydrierung der im CC4 enthaltenen mehrfach ungesättigten Kohlenwasserstoffe erhalten. Dabei werden beispielsweise aus 1,3-Butadien hauptsächlich 1-Buten und 2-Butene gebildet.

Raffinat I wird erhalten durch Abtrennung von 1,3-Butadien und anderen mehrfach ungesättigten Kohlenwasserstoffen aus dem CC4. Dies kann nach bekannten Verfahren, beispielsweise durch Extraktivdestillation, erfolgen.

In der nachfolgenden Tabelle 1 sind typische Massenanteile der Hautkomponenten von CC4, HCC4 und Raffinat I angegeben.

**Tabelle 1: Typische Zusammensetzung von CC4, HCC4 und Raffinat I.**

| Komponente | CC4 (S) | HCC4 | Raffinat I |
|---|---|---|---|
| Isobutan | 0,6 - 6 | 0,6 - 6 | 1 - 10 |
| n-Butan | 0,5 - 8 | 0,5 - 10 | 0,8 - 13 |
| 1-Buten | 9 - 25 | 24 - 67 | 15 - 42 |
| Isobuten | 10 - 35 | 10 - 35 | 17 - 58 |
| 2-Butene | 4 - 20 | 14 - 48 | 6 - 33 |
| 1,3-Butadien | 25 - 70 | 0 - 1 | 0 - 1 |
| C1-C3-KWSt | 0 - 1 | 0 - 1 | 0 - 2 |

| | | | |
|---|---|---|---|
| Erläuterung CC4 (S): typisch für eine C4 Mischung, die aus dem Crack-C4 eines Steamcrackers (High Severity) ohne zusätzliche Moderation des Katalysators erhalten wird. KWSt: Kohlenwasserstoffe Alle Angaben in Massen-% | | | |

Die nach teilweiser oder vollständiger Abtrennung von Isobuten aus Raffinat I oder HCC4 erhaltene Mischung von C4-Kohlenwasserstoffen wird in der Regel als Raffinat II bezeichnet. Der Restgehalt an Isobuten im Raffinat II variiert mit den Verfahren der Isobuten-Abtrennung und beträgt normalerweise weniger als 5 Massen-%. Typische Verfahren zur Abtrennung des Isobutens sind neben der Ethersynthese (MTBE, ETBE) die TBA-Synthese und die Dimerisierung des Isobutens.

### Verfahrensschritt a): MTBE-Synthese

In Verfahrensschritt a) des Verfahrens wird Isobuten (I), das in einer Mischung mit anderen C4-Kohlernwasserstoffen enthalten ist, als Isobuten-haltige C4-Kohlenwasserstoffe (II) eingesetzt. Besonders bevorzugt werden im erfindungsgemäßen Verfahren als Isobuten-haltige C4-Kohlenwasserstoffe (II) HCC4, Raffinat I, Mischungen selbiger oder Mischungen selbiger mit Raffinat II Strömen eingesetzt. Die eingesetzten C4-Kohlenwasserstoffströme können bis zur Sättigung Wasser enthalten. Typisch ist ein Wassergehalt von 200 - 400 ppm.
Zur Entfernung von polaren Komponenten ist es zudem bevorzugt, die C4-Kohlenwasserstoffe vor dem Einsatz in Verfahrensschritt a) mit Wasser zu waschen, wonach die C4-Ströme wassergesättigt vorliegen. Heterogen enthaltenes Wasser wird bevorzugt, beispielsweise über einen Dekanter mit oder ohne koaleszenzfördernde Einbauten, vor Einsatz in Verfahrensschritt a) abgetrennt.

In Verfahrenschritt a) des erfindungsgemäßen Verfahrens wird das in den Isobuten-haltigen C4-Kohlenwasserstoffen (II) enthaltene Isobuten (I) mit Methanol (III) an saueren lonentauschern unter Erhalt einer MTBE- und TBA-haltigen Reaktionsmischung (IV) umgesetzt. Prinzipiell können hierzu alle bekannten Verfahren zur MTBE-Synthese eingesetzt werden, beispielsweise kann die MTBE-Synthese analog zur Beschreibung in DE 101 02 082 erfolgen.

Die MTBE-Synthese im Verfahrensschritt a) wird vorzugsweise in mindestens zwei, besonders bevorzugt in drei Festbettreaktoren durchgeführt. Als Reaktoren, in denen das Methanol (III) mit dem Isobuten (I) bis nahe an das thermodynamische Gleichgewicht umgesetzt wird, können herkömmliche Festbettreaktoren (Rohrbündelreaktoren, adiabatische Festbettreaktoren, Kreislaufreaktoren) eingesetzt werden. Sie können mit oder ohne partielle Rückführung, wobei gegebenenfalls der Rückführungsstrom gekühlt werden kann, betrieben werden. Aus dem jeweils letzten der Festbettreaktoren wird eine MTBE- und TBA-haltige Reaktionsmischung (IV) abgezogen, die als weitere Bestandteile unter anderem nicht umgesetzte C4-Kohlenwasserstoffe, 2-Methoxybutan, C8-Kohlenwasserstoffe, DME und Restmengen an Methanol enthält.

Der Umsatz des Isobutens erfolgt möglichst bis zur Einstellung des thermodynamischen Gleichgewichts aus MTBE, Methanol und Isobuten, wobei vorzugsweise ein Isobuten-Umsatz von größer 90, besonders bevorzugt größer 95 %, ganz besonders bevorzugt größer 98 % eingestellt bzw. erreicht wird. Die Reaktoren werden vorzugsweise bei einer Temperatur von 20 bis 110 °C, bevorzugt 25 bis 70 °C und einem Druck von 0,5 bis 5 MPa, vorzugsweise 0,7 bis 2 MPa(abs) betrieben.

Da das thermodynamische Gleichgewicht zwischen Methanol/Isobuten und Ether bei tiefer Temperatur überwiegend auf der Seite des Ethers liegt, ist es bevorzugt, den ersten der Reaktoren bei höherer Temperatur zwecks hoher Reaktionsgeschwindigkeit als die Folgenden, in denen der Gleichgewichtslage ausgenutzt wird, zu betreiben. Vorzugsweise wird der erste der Reaktoren bei einer Temperatur von 35 bis 70 °C betrieben und die nachfolgenden Reaktoren werden bei einer Temperatur von 25 bis 50 °C betrieben.

Das Methanol (III) wird der Reaktion in Schritt a) über einen oder mehrere Methanolhaltige Ströme zugeführt, die noch weitere Komponenten enthalten können. Beispielsweise werden folgende Methanol-haltige Ströme verwertet: frisches, also nicht im erfindungsgemäßen Prozess zurückgewonnenes, Methanol (zum Ausgleich von Methanol-Verlusten durch MTBE-Ausschleusung oder DME-Bildung), die Methanol-haltige Fraktion (VIII) und Ströme, die bei der Fraktionierung von Wasser und Methanol aus Extraktionen anfallen. Methanol (III) bezieht sich im Rahmen dieser Erfindung auf die Summe des in diesen Strömen enthaltenen reinen Methanols.

Als weitere Komponenten können in diesen Strömen beispielsweise MTBE, TBA, Wasser, C8-Kohlenwasserstoffe und Isopren enthalten sein.

Es ist natürlich möglich, diese Ströme getrennt an verschiedenen Stellen des Verfahrens einzuspeisen. Bevorzugt ist jedoch, sie zu mischen und als einen gemeinsamen Strom zu nutzen.

Das molare Verhältnis von Methanol (III) zu Isobuten im Zulauf zum ersten Reaktor des Verfahrensschrittes a) liegt vorzugsweise im Bereich von 10 zu 1 bis 1 zu 1, besonders bevorzugt von 5 zu 1 bis 1,1 zu 1 und ganz besonders bevorzugt im Bereich von 1,8 zu 1 bis 1,2 zu 1.

Als Katalysatoren werden in der MTBE-Synthese feste saure lonenaustauscherharze, die Sulfonsäuregruppen aufweisen, eingesetzt. Geeignete lonenaustauscherharze sind beispielsweise solche, die durch Sulfonierung von Phenol/Aldehyd-Kondensaten oder von Cooligomeren von aromatischen Vinylverbindungen hergestellt werden. Beispiele für aromatische Vinylverbindungen zur Herstellung der Cooligomere sind: Styrol, Vinyltoluol, Vinylnaphthalin, Vinylethylbenzol, Methylstyrol, Vinylchlorbenzol, Vinylxylol und Divinylbenzol. Insbesondere werden die Cooligomeren, die durch Umsetzung von Styrol mit Divinylbenzol entstehen, als Vorstufe für die Herstellung von lonenaustauscherharzen mit Sulfonsäuregruppen verwendet. Die Harze können gelförmig, makroporös oder schwammförmig hergestellt werden. Die Eigenschaften dieser Harze, insbesondere spezifische Oberfläche, Porosität, Stabilität, Quellung bzw. Schrumpfung und Austauschkapazität, können durch den Herstellprozess variiert werden.

Das Porenvolumen der als Katalysatoren eingesetzten lonenaustauscherharze beträgt vorzugsweise von 0,3 bis 0,9 ml/g, insbesondere von 0,5 bis 0,9 ml/g. Die Korngröße der Harze beträgt bevorzugt von 0,3 mm bis 1,5 mm, insbesondere von 0,5 mm bis 1,0 mm. Die Korngrößenverteilung kann enger oder weiter gewählt werden. So können beispielsweise lonenaustauscherharze mit sehr einheitlicher Korngröße (monodisperse Harze) eingesetzt werden. Die Kapazität des lonenaustauscher beträgt, bezogen auf die Lieferform, bevorzugt von 0,7 bis 2,0 eq/l, insbesondere von 1,1 bis 2,0 eq/l bzw. vorzugsweise von 0,5 bis 5,5 mol/kg, insbesondere von 0,8 bis 5,5 mol/kg. Die Angaben zur Kapazität in mol/kg beziehen sich auf das jeweils bis zur Gewichtskonstanz im warmen Stickstoffstrom bei z. B. 105 °C getrocknete lonentauscherharz.

Im erfindungsgemäßen Verfahren können die lonenaustauscherharze in ihrer H-Form eingesetzt werden. Stark saure Harze des Styrol-Divinylbenzol-Typs, die vorzugsweise eingesetzt werden, werden u. a. unter folgenden Handelsnamen verkauft: Amberlyst® 15, Amberlyst® 35 (jeweils Rohm & Haas) oder Lewatit® K2621 (Lanxess).

### Verfahrensschritt b): MTBE-Abtrennung

Die destillative Abtrennung der MTBE- und TBA-haltigen Fraktion (V) aus der Reaktionsmischung (IV) kann im einfachsten Fall in einer einzigen Kolonne erfolgen. Dabei wird die Fraktion (V) als Sumpfprodukt erhalten. Das Destillat besteht hauptsächlich aus nicht umgesetzten C4-Kohlenwasserstoffen, die aufgrund der Azeotropbildung mit Methanol auch Methanol enthalten, und DME. Das Sumpfprodukt enthält neben TBA und MTBE noch 2-Methoxybutan und C8-Kohlenwasserstoffe. Abhängig vom gewählten Verhältnis Methanol (III) / Isobuten (I) und dem erreichten Umsatz kann auch noch Methanol enthalten sein.

Die Destillationskolonne weist vorzugsweise 15 bis 55 theoretische Trennstufen auf, bevorzugt 20 bis 40 und besonders bevorzugt 25 bis 35. Der Zulauf der Kolonne wird vorzugsweise zwischen Stufe 10 und 30 (von oben) zugegeben, bevorzugt zwischen Stufe 15 und 25. Die Kolonne wird vorzugsweise bei einem Druck von 0,3 bis 2,5 MPa(abs), bevorzugt bei 0,5 bis 1,0 MPa(abs), betrieben. Das Rücklaufverhältnis beträgt, in Abhängigkeit von der realisierten Stufenzahl, der Zusammensetzung des Reaktoraustrags und der erforderlichen Reinheiten von Destillat und Sumpfprodukt, vorzugsweise kleiner 5, bevorzugt kleiner 2. Als Rücklaufverhältnis bezeichnet man definitionsgemäß das Verhältnis des Rücklaufstromes in die Kolonne zum abgeführten Destillatstrom.

In einer bevorzugten Ausführungsform **AFF1** wir bei der Kolonne die MTBE- und TBA-haltigen Fraktion (V) als Seitenabzug entnommen. Als Sumpfprodukt fällt eine Fraktion an, in der C8-Kohlenwasserstoffe angereichert sind und die so aus dem Prozess ausgeschleust werden. Die Abtrennung von C8- und gegebenenfalls C8+-Kohlenwasserstoffen hat den Vorteil, dass kein oder nur sehr wenig hiervon in den Spaltungsreaktor gelangen. Dadurch wird der Katalysator in der MTBE-Spaltung vor einer Belegung mit Hochsiedern geschützt, wodurch die Gefahr der Aktivitätsminderung und der Verkürzung der Standdauer des Katalysators vermindert wird.

Eine Destillationskolonne, bei dem die MTBE- und TBA-haltigen Fraktion (V) als Seitenabzug entnommen, weist vorzugsweise 20 bis 65 theoretische Trennstufen auf, bevorzugt 25 bis 55 und besonders bevorzugt 30 bis 45. Der Zulauf der Kolonne wird vorzugsweise zwischen Stufe 10 und 30 (von oben) zugegeben, bevorzugt zwischen Stufe 15 und 25. Die Entnahme des Seitenstroms erfolgt vorzugsweise unterhalb der Zugabe des Zulaufs, bevorzugt zwischen 5 und 25, besonders bevorzugt zwischen 10 und 20 Stufen unterhalb des Zulaufes und vorzugesweise zwischen 5 und 20 Stufen oberhalb der Sumpfentnahme, bevorzugt 10 und 15 Stufen oberhalb. Die Kolonne wird vorzugsweise bei einem Druck von 0,3 bis 2,5 MPa(abs), bevorzugt bei 0,5 bis 1,0 MPa(abs), betrieben. Das Rücklaufverhältnis beträgt, in Abhängigkeit von der realisierten Stufenzahl, der Zusammensetzung des Reaktoraustrags und der erforderlichen Reinheiten von Destillat und Sumpfprodukt, vorzugsweise kleiner 5, bevorzugt kleiner 2.

In einer weitern bevorzugten Ausführungsform **AFF2** des Verfahrensschrittes b) wird die Kolonne als Reaktivdestillation ausgeführt. Dies hat den Vorteil, dass zusätzliches in Verfahrensschritt a) nicht umgesetztes Isobuten zu MTBE umgesetzt wird.

Die Reaktivdestillationskolonne wird in einem Druckbereich von 0,3 bis 2,5 MPa(abs), vorzugsweise 0,5 bis 1,0 MPa(abs) und bei einer Temperatur in der Reaktionszone von 50 bis 90 °C, vorzugsweise 55 bis 70 °C bei einem Rücklaufverhältnis zwischen 0,5 und 1,5, bevorzugt zwischen 0,7 und 0,9 betrieben.

Bevorzugt weist die Reaktivdestillationskolonne oberhalb der Katalysatorpackung einen Bereich rein destillativer Trennung auf. Bevorzugt weist die Zone oberhalb der Katalysatorpackung 5 bis 25, insbesondere 10 bis 15 Trennstufen auf. Die Trennzone unterhalb des Katalysators umfasst 10 bis 40, insbesondere 20 bis 30 Trennstufen.

Der Zulauf zur Reaktivdestillationskolonne kann oberhalb oder unterhalb, vorzugsweise unterhalb der Katalysatorzone erfolgen. Der Zulauf zur Reaktivdestillationskolonne erfolgt vorzugsweise unterhalb der reaktiven Packung, bevorzugt 3 bis 13, besonders bevorzugt 4 bis 10 theoretische Trennstufen unterhalb der reaktiven Packung.

Die Katalysatorzone kann mit einer destillativen Wirkung von 1 bis 5 theoretischer Trennstufen pro Meter Packungshöhe abgeschätzt werden. Die Höhe der Katalysatorzone/Reaktivzone lässt sich in Abhängigkeit vom gewünschten Isobuten-Umsatz durch einfache Vorversuche ermitteln. Die Katalysatormenge wird vorzugsweise so groß gewählt, dass ein Isobuten-Umsatz von 75 bis 99 %, vorzugsweise von 85 bis 98 % und besonders bevorzugt von 95 bis 97 % bezogen auf den Isobutengehalt im Zulauf zur Reaktivdestillation erreicht wird.

Als Katalysator wird wie in der Verfahrensstufe a) ein saures lonentauscherharz verwendet. In der Reaktivdestillationskolonne kann der Katalysator entweder in der Packung integriert sein, beispielsweise KataMax® (wie in EP 0 428 265 beschrieben), KataPak® (wie in EP 0 396 650 oder DE 298 07 007.3 U1 beschrieben) oder auf Formkörpern aufpolymerisiert (wie in US 5,244,929 beschrieben) sein.

Die hydraulische Belastung in der katalytischen Packung der Kolonne beträgt bevorzugt 10 % bis 110 %, vorzugsweise 20 % bis 70 % ihrer Flutpunktbelastung. Unter hydraulischer Belastung einer Destillationskolonne wird die gleichmäßige strömungstechnische Beanspruchung des Kolonnenquerschnitts durch den aufsteigenden Dampf-Massenstrom und den rücklaufenden Flüssigkeits-Massenstrom verstanden. Die obere Belastungsgrenze kennzeichnet die maximale Belastung durch Dampf und Rücklaufflüssigkeit, oberhalb derer die Trennwirkung infolge Mitreißens oder Stauens der Rücklaufflüssigkeit durch den aufsteigenden Dampfstrom absinkt. Die untere Belastungsgrenze kennzeichnet die minimale Belastung, unterhalb derer die Trennwirkung absinkt oder zusammenbricht infolge unregelmäßiger Strömung oder Leerlaufen der Kolonne - z. B. der Böden. (Vauck/Müller, "Grundoperationen chemischer Verfahrenstechnik", S. 626, VEB Deutscher Verlag für Grundstoffindustrie.)

Am Flutpunkt werden die vom Gas an die Flüssigkeit übertragenen Schubspannungen so groß, dass die gesamte Flüssigkeitsmenge in Form von Tropfen mit dem Gas mitgerissen wird oder dass es zu Phaseninversion in der Kolonne kommt (J. Mackowiak, "Fluiddynamik von Kolonnen mit modernen Füllkörpern und Packungen für Gas/Flüssigkeitssysteme", Otto Salle Verlag 1991).

Durch die Reaktivdestillationstechnik zum weitern Isobuten-Umsatz können insbesondere Isobuten-Restkonzentrationen von kleiner 1000 Massen-ppm (wppm), bevorzugt 800 wppm und besonders bevorzugt kleiner 500 wppm, bezogen auf das C4-Gemisch im Destillat, erhalten werden.

Im Kolonnenzulauf kann mehr Methanol enthalten sein, als für die vollständige Umsetzung des noch vorhandenen Isobutens gebraucht wird. Der Methanolüberschuss sollte jedoch derart begrenzt werden, dass einerseits eine ausreichende Methanolmenge für das sich bildende Azeotrop aus Methanol und C4-Kohlenwasserstoffen vorhanden ist, andererseits nicht so viel, dass übermäßig viel Methanol ins Sumpfprodukt geriete, so dass bevorzugt ein MTBE mit einem Methanolgehalt unter 10000 wppm, besonders bevorzugt unter 5000 wppm erhalten wird.

Optional kann zusätzliches Methanol in die Reaktivdestillationskolonne eingespeist werden. Dies kann zusammen mit dem Zulauf aus Verfahrensschritt a) oder aber auch an einer Stelle oder an mehreren Stellen der Reaktivdestillationskolonne erfolgen, z. B. am Kolonnenkopf und/oder auf, zwischen und/oder unter dem Katalysatorbett.

Die als Destillat abgetrennten Methanol-haltigen C4-Kohlenwasserstoffe können in verschiedenen Aufarbeitungsvarianten weiterverarbeitet werden.

In einer bevorzugten Aufarbeitungsvariante wird aus den als Destillat erhaltenen Methanol-haltigen C4-Kohlenwasserstoffen das Methanol abgetrennt. Die Abtrennung des Methanols aus dem Kopfprodukt kann insbesondere durch Extraktion mit Wasser oder einer wässrigen Lösung als Waschmedium erfolgen. Bevorzugt wird eine wässrige Lösung mit einem pH-Wert von größer-gleich 8, bevorzugt von 8 bis 12 eingesetzt. Die Einstellung des pH-Werts kann z. B. durch Zugabe von Natronlauge und/oder Schwefelsäure erfolgen. Diese Extraktion nach den bekannten Standardverfahren der Technik, kann beispielsweise in einer Extraktionskolonne oder in einer Kaskade von Mixern und Trennbehältern erfolgen. Bevorzugt erfolgt die Abtrennung des Methanols mit Wasser oder einer wässrigen Lösung als Waschmedium in einer Extraktionskolonne. Der Restgehalt an Alkohol wird dabei bevorzugt auf unter 0,2 Massen-%, besonders bevorzugt auf unter 500 wppm, ganz besonders bevorzugt auf unter 50 wppm abgesenkt. Die Extraktionskolonne weist bevorzugt von 2 bis 25, besonders bevorzugt von 5 bis 15 theoretische Trennstufen auf und wird bevorzugt bei Temperaturen von 10 bis 90 °C und Drücken von mindestens 0,1 MPa über dem Dampfdruck der C4-Kohlenwasserstoffe betrieben. Das Massen-Verhältnis von Waschmedium zu zugeführtem Kopfprodukt (technische Mischung IV plus Methanol) beträgt vorzugsweise von 1 zu 5 bis 1 zu 40.

Das mit Alkohol beladene Waschwasser aus der Extraktion wird bevorzugt in einer separaten Einheit aufgearbeitet und das Wasser zumindest teilweise in die Extraktion zurückgeführt. Die Aufarbeitung kann beispielsweise durch eine Destillation erfolgen, bei der eine praktisch alkoholfreie Wasserfraktion im Sumpf und Methanol als Kopfprodukt erhalten werden. Das Methanol kann zurück in den Schritt a) des erfindungsgemäßen Verfahrens gefahren werden.

Die aus der Extraktion erhaltenen Methanol-freien C4-Kohlenwaserstoffe können nach bekannten Verfahren zu hochreinem 1-Buten aufgearbeitet werden. Hierzu werden beispielsweise zuerst in einer Selektiven Hydrierung Spuren mehrfach ungesättigter Kohlenwasserstoffe hydriert (z.B. DE 31 43 647) und anschließend durch zweimalige Fraktionierung zuerst n-Butan und 2-Butene, danach Isobutan und weitere Leichtsieder vom 1-Buten abgetrennt. Die bei der ersten Fraktionierung abgetrennten 2-Butene und das n-Butan können beispielsweise in einer Oligomerisierung zu C8-Olefinen (z. B. OCTOL-Prozess, s. Hydrocarbon Process, Int. Ed. (1986)65, S. 31 - 33) oder in einer Hydroformylierung zu Valeraldehyd weiterverarbeitet werden.

In einer zweiten bevorzugten Aufarbeitungsvariante für die abgetrennten C4-Kohlenwasserstoffe werden diese zuerst einer weitern Reaktionsstufe zugeführt, um den Isobutengehalt weiter zu verringern. Diese Umsetzung erfolgt bevorzugt in einem oder zwei adiabatischen Festbettreaktoren, als Katalysatoren kommen die gleichen wie bei Verfahrensstufe a) zum Einsatz. Die Temperaturen, bei der die Umsetzung durchgeführt wird, liegen vorzugsweise bei 20 bis 60 °C, bevorzugt bei 30 - 50 °C, der Druck vorzugsweise bei 0,5 bis 5 MPa, bevorzugt 0,7 bis 2 MPa. Bevorzugt wird in die Reaktionsstufe zusätzliches Methanol eingespeist, so dass der Methanol-Gehalt am Ausgang der Reaktoren bei bevorzugt 1 - 10 Massen-% liegt. Der Austrag der Reaktionsstufe wird in einer Destillationskolonne getrennt, wobei als Destillat Methanol-haltige C4-Kohlenwasserstoffe erhalten werden. Auch diese Destillation kann als Reaktivdestillation ausgeführt werden. Die C4-Kohlenwasserstoffe können analog zur ersten Aufarbeitungsvariante durch Extraktion, Selektivhydrierung und Destillation weiter verarbeitet werden. Als Sumpfprodukt fällt ein MTBE-Strom an, der normalerweise noch Methanol enthält. Er kann separat verwertet oder ganz oder teilweise in Verfahrensschritt a) zurückgeführt werden.

Bei Aufarbeitung nach dieser zweiten Aufarbeitungsvariante kann die destillative Trennung der Reaktionsmischung (IV) mit geringerem Aufwand betrieben werden, so dass noch Anteile an MTBE im Destillatstrom verbleiben. Dadurch lässt sich der notwendige Energieeinsatz reduzieren.

In einer weitern bevorzugten Ausführungsform AFF3 des Verfahrensschrittes b) wird nach der ersten Kolonne zur Trennung der Reaktionsmischung (IV) das MTBE-Sumpfprodukt der ersten Kolonne in einer zweiten Kolonne weiter aufgereinigt. In dieser Kolonne werden Schwersieder, vor allen C8-Kohlenwasserstoffe, als Sumpfprodukt entfernt. Eine weitere Aufgabe dieser Kolonne kann die teilweise oder vollständige Abtrennung von 2-Methoxybutan sein, denn 2-Methoxybutan kann im Reaktor zu linearen Butenen und Methanol gespalten werden. Lineare Butene können bei zu hoher Konzentration gegebenenfalls die Isobuten-Spezifikation gefährden.

Die Kolonne wird bei einem Druck betrieben, der möglichst den Großteil des im Zulauf enthaltenen TBAs ins Destillat gelangen lässt (Ausbildung eines Druckazeotrops). Der Kolonnendruck liegt daher, unabhängig davon, ob nur DIB oder zusätzlich 2-Methoxybutan abgetrennt werden soll, vorzugsweise bei 0,3 bis 2,0 MPa_{(abs)}. Wenn die Spaltung der am Kolonnenkopf erhaltenen MTBE- und TBA-haltigen Fraktion (V) im Spaltreaktor in der Gasphase bei erhöhtem Druck erfolgt, kann es vorteilhaft sein, die Destillation bei höherem Druck durchzuführen, wobei in diesem Fall der Kopfkondensator vorzugsweise als Partialkondensator betrieben wird und das Kopfprodukt (V) dampfförmig abgezogen wird. Das dampfförmig abgezogene Kopfprodukt kann dann direkt oder nach weiterer Vorwärmung dem Reaktor zugeführt werden. Der Druckunterschied zwischen Destillation und Reaktor beträgt hierbei bevorzugt mindestens 0,05 MPa_{(abs)}. Beträgt der Reaktionsdruck im Spaltreaktor beispielsweise 0,7 MPa_{(abs)}, so sollte der Destillationsdruck vorzugsweise mindestens 0,75 MPa_{(abs)} betragen. Bei Betriebsdrucken von größer 0,95 MPa_{(abs)} kann mit der Kondensationswärme (Niederdruck-) Dampf erzeugt werden, mit dem andere Kolonnen des Verfahrens beheizt werden können. Zur Beheizung der Kolonne kann, je nach gewähltem Betriebsdruck, Dampf oder Wärmeträgeröl eingesetzt werden.

Wenn in der Kolonne insbesondere nur C8-Kohlenwasserstoffe abgetrennt werden sollen, kann es vorteilhaft sein, wenn die Kolonne 15 bis 60 theoretische Trennstufen, vorzugsweise 20 bis 55 und bevorzugt 30 bis 45 theoretische Trennstufen aufweist. Das Rücklaufverhältnis, im Rahmen der vorliegenden Erfindung definiert als der Massenstrom des Rücklaufs geteilt durch den Massenstrom des Destillats, wird, in Abhängigkeit von der realisierten Stufenzahl, der Zusammensetzung des eingesetzten MTBE und der erforderlichen Reinheit, vorzugsweise auf einen Wert von 0,5 bis 7, bevorzugt von 1 bis 4 eingestellt.

Soll in der Kolonne DIB und zusätzlich 2-Methoxybutan abgetrennt werden, weist die eingesetzte Destillationskolonne vorzugsweise von 50 bis 140 theoretische Trennstufen, bevorzugt von 60 bis 120 und ganz besonders bevorzugt von 80 bis 110 auf. Das Rücklaufverhältnis beträgt, in Abhängigkeit von der realisierten Stufenzahl, der Zusammensetzung des eingesetzten MTBE und der erforderlichen Reinheit, vorzugsweise von 1 bis 20, bevorzugt von 3 bis 10.
Selbst wenn die Abtrennung von 2-Methoxybutan nicht notwendig sein sollte, muss die Auslegung der Kolonne mit höherer Bodenzahl kein Nachteil sein, da ein Teil des höheren Kapitaleinsatzes für die größere Kolonne durch Energieeinsparung (Verringerung des Rücklaufverhältnisses) kompensiert werden kann. Gleichzeit erhält man hierdurch eine höhere operative Flexibilität.

Das Sumpfprodukt der Kolonne enthält die Hochsieder Diisobuten und 2-Methoxybutan sowie MTBE. Soll in der Kolonne vornehmlich Diisobuten abgetrennt werden, kann der MTBE-Gehalt im Sumpfprodukt auf Werte kleiner 25 Massen-% reduziert werden. Soll zusätzlich 2-Methoxybutan abgetrennt werden, wird man aufgrund der kleinen Siedepunktsunterschiede zwischen 2-Methoxybutan und MTBE zweckmäßigerweise einen höheren MTBE-Gehalt im Sumpfprodukt zwischen 60 und 85 Massen-% zulassen, um den Aufwand für die Trennung zu reduzieren.
In beiden Fällen kann dieses Gemisch thermisch verwertet werden, als Einsatzstoff für eine Synthesegasanlage genutzt werden oder direkt oder nach Hydrierung als Treibstoffkomponente verwendet werden.

In einer weitern bevorzugten Ausführungsform AFF4 des Verfahrensschrittes b) wird zwischen die beiden Kolonnen der AFF3 eine weitere Destillationskolonne zur Abtrennung von Nebenprodukten geschaltet. In dieser Kolonne werden über Kopf mittelsiedende Komponenten ganz oder teilweise als Destillat abgetrennt. Als mittelsiedende Komponenten werden im Rahmen dieser Erfindung Komponenten mit einem Siedpunkt zwischen den C4-Kohlenwasserstoffen und MTBE bzw. dem MTBE/Methanol-Azeotrop verstanden. Beispiel hierfür sind Dimethoxymethan und C5-Kohlenwasserstoffe wie n-Pentan, Isopentan, Neopentan, 1-Penten, 2-Penten, 3-Methyl-1-buten und Isopren. Reste an gegebenenfalls noch enthaltenen C4-Kohlenwasserstoffen werden zusammen mit den mittelsiedenden Komponenten abgetrennt. Der Anteil an mittelsiedenden Komponenten im Zulauf zu der Kolonne ist in der Regel nicht hoch und liegt vorzugsweise unter 2 %, bevorzugt unter 0,5 %.

Die Alternative zur Abtrennung der mittelsiedenden Komponenten wäre eine Abtrennung zusammen mit der in der Regel viel größeren Menge an in der C4-Kohlenwasserstoffen über das Destillat der ersten Kolonne. Aus diesen wären sie aber destillativ nur als Sumpfprodukt abtrennbar. Da hierzu die gesamten C4-Kohlenwasserstoffe über Kopf destilliert werden müssten, wäre diese Option energetisch ungünstiger.

Vorzugsweise weist die Destillationskolonne zur Abtrennung der mittelsiedenden Komponenten 30 bis 75 theoretische Trennstufen, bevorzugt 40 bis 65 und besonders bevorzugt 40 bis 55 theoretische Trennstufen auf. Vorzugsweise wird die Kolonne, in Abhängigkeit von der realisierten Stufenzahl, der Zusammensetzung des eingesetzten MTBE und der erforderlichen Reinheit an mittelsiedenden Komponenten mit einem Rücklaufverhältnis zwischen 150 und 350, insbesondere zwischen 200 und 300 betrieben. Die Kolonne wird vorzugsweise mit einem Betriebsdruck von 0,2 bis 0,6 MPa_{(abs)}, bevorzugt von 0,3 bis 0,4 MPa_{(abs)} betrieben. Zur Beheizung der Kolonne kann z. B. Dampf eingesetzt werden. Die Kondensation kann, je nach gewähltem Betriebsdruck, gegen Kühlsohle, Kühlwasser oder Luft erfolgen. Der Kopfbrüden der Kolonne können vollständig oder nur teilweise kondensiert werden, so dass das Kopfprodukt entweder flüssig oder dampfförmig abgezogen werden kann. Das Kopfprodukt kann beispielsweise thermisch verwertet oder als Einsatzstoff einer Synthesegasanlage genutzt werden.

Die in Verfahrenschritt b) abgetrennte MTBE- und TBA-haltige Fraktion (V) weist vorzugsweise einen Gehalt an MTBE von größer 95 Massen-%, besonders bevorzugt größer 98 Massen-% auf. Der Anteil an TBA liegt vorzugsweise bei 0,05 bis 5 Massen-%, bevorzugt bei 0,2 bis 2,0 Massen-%, ganz besonders bevorzugt bei 0,2 bis 1,0 Massen-%. Neben MTBE und TBA kann Methanol enthalten sein, vorzugsweise 0 bis 5 Massen-%, bevorzugt 0,1 bis 1 Massen-%.

Konzentration und Auftreten weiterer Komponenten sind stark abhängig von den in Verfahrensschritt a) eingesetzten Rohstoffen und der Verfahrensführung in den Schritten a) und b). Der Gehalt an 2-Methoxybutan liegt vorzugsweise bei 0 bis 1,0 Massen-%, bevorzugt bei 0,05 bis 0,5 Massen-%, besonders bevorzugt bei 0,08 bis 0,3 Massen-%. Der Gehalt an C8-Kohlenwasserstoffen liegt vorzugsweise bei 0 bis 1 Massen-%, bevorzugt bei 0 bis 0,1 Massen-%, besonders bevorzugt bei 0 bis 0,01 Massen-%.

Typische andere enthaltene Komponenten sind 3-Methoxy-1-buten, 1-Methoxy-2-buten, Dimethoxymethan und C5-Kohlenwasserstoffe.

### Verfahrensschrit c): MTBE-Spaltung

In Schritt c) des erfindungsgemäßen Verfahrens wir die MTBE- und TBA-haltige Fraktion (V) vollständig oder teilweise einer Spaltungsreaktion zugeführt, wo sie an einem heterogenen Katalysator in der Gasphase zu mindestens Isobuten, Methanol, MTBE und Wasser und gegebenenfalls TBA enthaltenden Spaltprodukten (VI) gespalten wird. Dabei können alle festen Katalysatoren eingesetzt werden, die im Temperaturbereich 120 bis 400 °C, insbesondere im Bereich von 180 bis 350 °C, die Spaltung von MTBE zu Isobuten und Methanol bewirken.

Bevorzugt wird das erfindungsgemäße Verfahren so ausgeführt, dass ein möglichst großer Teil des in Schritt a) erzeugten MTBEs dem Schritt c) zugeführt wird. Es eignet sich daher insbesondere zur Produktion von reinem Isobuten aus Isobuten-haltigen C4-Kohlenwasserstoffen, wenn ein hoher Anteil des in den C4-Kohlenwasserstoffen enthaltenen Isobutens als reines Isobuten erhalten werden soll. Alternativ können die im Verfahren auftretenden MTBE-reichen Ströme anteilig anders, beispielsweise zur Nutzung als Kraftstoffzusatz, eingesetzt werden. Besonders bevorzugt ist es aber, mindestens 80 Massen-%, ganz besonders mindestens 90 Massen-% des in der Reaktionsmischung (IV) enthaltenen MTBEs über die Fraktion (V) dem Schritt c) zuzuführen.

Die im erfindungsgemäßen Verfahren eingesetzten Katalysatoren können z. B. Metalloxide, Metallmischoxide, insbesondere solche, die Siliziumoxid und/oder Aluminiumoxid enthalten, Säuren auf Metalloxidträgern oder Metallsalze oder Mischungen davon sein.

Im erfindungsgemäßen Verfahren werden zur Spaltung von MTBE zu Isobuten und Methanol in der Gasphase bevorzugt Katalysatoren verwendet, die formal aus Magnesiumoxid, Aluminiumoxid und Siliziumoxid bestehen. Solche Katalysatoren werden z. B. in US 5,171,920 in Beispiel 4 oder in EP 0 589 557 beschrieben.

Besonders bevorzugt werden Katalysatoren eingesetzt, die formal Magnesiumoxid, Aluminiumoxid und Siliziumdioxid aufweisen, und die einen Anteil an Magnesiumoxid von 0,5 bis 20 Massen-%, bevorzugt von 5 bis 15 Massen-% und besonders bevorzugt von 10 bis 15 Massen-%, einen Anteil an Aluminiumoxid von 4 bis 30 Massen-%, bevorzugt von 10 bis 20 Massen-% und einen Anteil an Siliziumdioxid von 60 bis 95 Massen-%, bevorzugt von 70 bis 90 Massen-% aufweisen. Es kann vorteilhaft sein, wenn der Katalysator neben dem Magnesiumoxid ein Alkalimetalloxid aufweist. Dieses kann z. B. ausgewählt sein aus Na₂O oder K₂O. Vorzugsweise weist der Katalysator als Alkalimetalloxid Na₂O auf. Der bevorzugt eingesetzte Katalysator weist vorzugsweise eine BET-Oberfläche (volumetrisch bestimmt mit Stickstoff gemäß DIN ISO 9277) von 200 bis 450 m²/g, bevorzugt von 200 bis 350 m²/g auf. Wird der erfindungsgemäße Katalysator als aktive Masse auf einem Träger aufgebracht, so weist nur die aktive Masse eine BET-Oberfläche im genannten Bereich auf. Das Material aus Katalysator und Träger kann dagegen je nach Beschaffenheit des Trägers eine deutlich abweichende BET-Oberfläche, insbesondere eine kleinere BET-Oberfläche aufweisen.

Das Porenvolumen des Katalysators beträgt vorzugsweise von 0,5 bis 1,3 ml/g, bevorzugt von 0,65 bis 1,1 ml/g.

Der mittlere Porendurchmesser (vorzugsweise bestimmt in Anlehnung an DIN 66133) des Katalysators beträgt vorzugsweise von 5 bis 20 nm, bevorzugt von 8 bis 15 nm. Besonders bevorzugt entfällt mindestens 50 %, vorzugsweise über 70 % des Gesamtporenvolumens (Summe des Porenvolumens der Poren mit einem Porendurchmesser von größer-gleich 3,5 nm bestimmt mit Quecksilberporosimetrie gemäß DIN 66133) des Katalysators auf Poren mit einem Durchmesser von 3,5 bis 50 nm (Mesoporen).

Im erfindungsgemäßen Verfahren werden bevorzugt Katalysatoren eingesetzt, die eine mittlere Korngröße (bestimmt durch Siebanalyse) von 10 µm bis 10 mm, vorzugsweise 0,5 mm bis 10 mm, besonders bevorzugt eine mittlere Korngröße von 1 bis 5 mm aufweisen. Vorzugsweise werden feste Katalysatoren eingesetzt, die eine mittlere Korngröße d₅₀, von 2 bis 4 mm, insbesondere von 3 bis 4 mm.

In dem erfindungsgemäßen Verfahren kann der Katalysator als Formkörper eingesetzt werden. Die Formkörper können jegliche Form einnehmen. Bevorzugt wird der Katalysator als Formköper in Form von Kugeln, Extrudaten oder Tabletten eingesetzt. Die Formkörper weisen vorzugsweise die oben genannten mittleren Korngrößen auf.

Die Herstellung und Verwendung solcher Magnesium-Alumosilikat-Katalysatoren ist in DE 10 2006 040432.7 beschrieben. Es wird darauf Bezug genommen.

Es kann für die Aktivität und / oder Selektivität einiger Katalysatoren vorteilhaft sein, wenn dem Reaktorzulauf Anteile an Wasser zugegeben werden. So beschreibt beispielsweise JP 19912201 die kontinuierliche Zugabe von Wasser zu einem Alkalioder Erdalkalimetall-dotiertem Alumosilikat zur Verringerung der Bildung von Nebenkomponenten.

Im erfindungsgemäßen Verfahren kann die Zugabe von Wasser zum Reaktorzulauf beispielsweise durch Zugabe von Wasser in den Strom (V) erfolgen. Dies erfolgt beispielsweise direkt durch Zugabe von Wasser oder Wasserdampf in den gasförmigen Strom (V), der dem Reaktor zugeleitet wird. Wird der Strom (V) vor dem Reaktor separat verdampft, kann hier Wasser zugegeben werden.
Eine weitere Möglichkeit besteht darin, das Wasser schon im Verfahrensschritt b) bei der Abtrennung der MTBE- und TBA-haltigen Fraktion (V) zuzugeben. Bevorzugt wird dabei das Wasser in den Kolonnenzulauf oder in den Rücklauf der Kolonne eingespeist. Besonders bevorzugt erfolgt dies in der Kolonne, in der Strom (V) abgetrennt wird.

Die optionale Zugabe von Wasser zur Moderation des Katalysators erfolgt derart, dass der Wasseranteil in Strom (V) bevorzugt 0 bis 5 Massen-%, besonders bevorzugt 0,2 bis 1,5 Massen-% beträgt.
Als zugeführtes Wasser wird dabei bevorzugt vollständig demineralisiertes oder destilliertes Wasser oder Wasserdampf eingesetzt.

Die Spaltung des MTBE wird in der Gasphase im Temperaturbereich von 120 bis 400 °C, insbesondere 180 bis 350 °C bei Drücken von 0,1 bis 2 MPa(abs), insbesondere bei Drücken von 0,3 bis 1 MPa(abs), ganz besonders bei Drücken von 0,5 bis 0,8 MPa(abs).

Die Spaltung von MTBE in Isobuten und Methanol ist eine endotherme Reaktion, dass bedeutet, dass sich die Reaktionsmischung bei der Reaktion abkühlt. Bevorzugt wird der Reaktor derart betrieben, dass es unter den gewählten Druckbedingungen zu keiner Partialkondensation von MTBE und Produkten am Katalysator kommt. Besonders bevorzugt wird der Reaktor so betrieben, dass die Minimaltemperatur im Reaktor an jeder Stelle des Katalysatorbetts größer als 150 °C, ganz besonders bevorzugt größer 200 °C ist. Der maximale Temperaturabfall kann durch zahlreiche Parameter, wie z. B. durch die Temperatur des zum Heizen verwendeten Wärmeträgers sowie durch die Geschwindigkeit, mit der der Wärmträger durch den Mantel strömt, eingestellt werden. Bevorzugt wird das Temperaturprofil im Katalysatorbett durch eine ausreichende Anzahl von Temperaturmessungen überwacht.

Im Laufe des Betriebes kann es vorteilhaft sein, mit zunehmender Desaktivierung des Katalysators zur Konstanthaltung des Umsatzes die Eingangs- und / oder Betriebstemperatur anzuheben.

Der Umsatz des MTBEs in Schritt c) des erfindungsgemäßen Verfahrens beträgt zwischen 40 % und 98 %, bevorzugt zwischen 75 % und 97 %, besonders bevorzugt zwischen 85 % und 95 %.

TBA wird unter den Bedingungen für die Spaltung des MTBEs ebenfalls zumindest teilweise gespalten. Der Umsatz ist dabei limitiert durch die Gleichgewichtslage der Reaktion bei der entsprechenden Temperatur.

Der Reaktor wird vorzugsweise mit einer Raumgeschwindigkeit (Weight Hourly Space Velocity (WHSV) in Kilogramm Edukt je Kilogramm Katalysator je Stunde) von 0,1 bis 5 h⁻¹, insbesondere von 1 bis 3 h⁻¹ im geraden Durchgang betrieben.

Als Reaktoren werden bevorzugt Rohrreaktoren oder Rohrbündelreaktoren, insbesondere solche mit inneren Rohrdurchmessern von 10 bis 60 mm, verwendet. Die Beheizung der Rohre erfolgt über den Reaktormantel durch Dampf, Salzschmelzen oder Wärmeträgeröle. Insbesondere bei Einsatz von flüssigen Heizmedien wird dabei die Mantelseite konstruktiv so ausgeführt, dass eine möglichst homogener Temperaturgradient an allen Rohren anliegt. Die hierfür notwendigen technischen Maßnahmen sind dem Fachmann bekannt und in der Literatur beschrieben (Einbau von Umlenkblechen, Disc- an Donut-Bauweise, Einspeisung / Abfuhr des Wärmeträgers an verschiedenen Stellen des Reaktors usw.). Bevorzugt werden Reaktionsmedium und Wärmeträger im Gleichstrom, besonders bevorzugt von oben nach unten durch die Traktorrohre bzw. den Reaktormantel geführt. Eine bevorzugte Ausführungsform ist beispielsweise in DE 10 2006 040433.5 beschrieben.

In herkömmlichen Rohrbündelapparaten werden Umlenkbleche auf der Mantelseite eingesetzt, die die eigentlich parallel zu den Rohren verlaufende Strömung des Wärmeträgerfluids in eine Querströmung umlenken sollen. Auf diese Weise wird der Wärmeübergang verbessert und zusätzlich die Fließweglänge deutlich erhöht. Allerdings treten zwischen den Umlenkblechen und dem äußeren Mantel des Wärmeübertragers fertigungsbedingte Spalte auf. Um das Rohrbündel in den Mantel einschieben zu können, wird ein Spalt mit einer Spaltweite von einigen Millimetern (in der Regel größer als 3 mm) benötigt. Diese Spaltverluste führen unweigerlich zu einer Verringerung der übertragenen Wärme.

In den Spalten zwischen Mantel und den Umlenkblechen treten aufgrund der Druckdifferenzen zwischen den einzelnen Segmenten zwangsläufig Leckströmungen auf, das durch den Randspalt fließende Fluid nimmt praktisch nicht an der Wärmeübertragung teil. Insgesamt können die Leckströmungen bis zu ca. 40 % der Gesamtströmung durch den Mantelraum betragen.

Daher wird in einer besonders bevorzugten Ausführungsform des erfindungsgemäßen Verfahrens ein spezieller Reaktortyp eingesetzt, bei dem durch eine spezielle Konstruktion die in Rohrbündelapparaten auftretenden Leckströmungen zwischen den Umlenkblechen und dem Apparatemantel gänzlich verhindert wird und so der Wärmeübergang deutlich verbessert wird.

Bei der speziellen Konstruktion handelt es sich um einen weiteren (Blech-)Mantel, im Folgenden Hemd genannt, der um das Rohrbündel gelegt wird. Anders als der eigentliche Mantel wird dieses Hemd spaltlos an die Umlenkbleche angelegt und dort befestigt. Das Hemd ist spaltlos mit den Umlenkblechen und mit dem Rohrboden auf der Seite des mantelseitigen Fluideintritts verbunden. Das setzt in der Regel voraus, dass dieses Hemd aus kleineren Segmenten besteht und diese Segmente an den Umlenkblechen befestigt werden. Aus Fertigungsgründen werden diese Segmente mit den Umlenkblechen, aber auch miteinander, vorzugsweise verschweißt oder auf eine andere Weise befestigt. Durch diese Fertigungsweise muss das in der Regel recht lange Rohrbündel nicht in das Hemd eingefädelt werden, sodass auf Spalte gänzlich verzichtet werden kann.

Im Betrieb wird der Mantelraum nur innerhalb des vom Hemd umschlossenen Rohrbündels durchströmt. Sinnvoller Weise ist das Hemd nicht über das letzte Umlenkblech hinaus um das Rohrbündel befestigt, sodass das durch den Mantelraum strömende Fluid das Hemd auf der vom Rohrbündel abgewandten Seite umschließt und so einen Druckausgleich bewirkt. Auf der Hemdkonstruktion lastet bei dieser Ausführung nur die Druckdifferenz, die aus den dynamischen Druckverlusten innerhalb des Rohrbündels resultiert. Die beschriebene Hemdkonstruktion ist daher nicht drucktragend, und kann deshalb im Vergleich zum äußeren Mantel mit eher geringen Wandstärken ausgeführt werden. Für das Hemd wird daher eine vorzugsweise dünnwandige Blechkonstruktion gewählt. Durch die Verwendung dünnwandiger Materialien, kann die Fertigung deutlich vereinfacht werden, und das Anlegen des Bleches an die Umlenkbleche wird wesentlich erleichtert.

Alternativ ist es denkbar, den äußeren Mantel auf die oben beschriebene Weise direkt mit den Umlenkblechen zu verschweißen. Im Vergleich zu einer solchen Lösung bietet die hier vorgestellte Hemdkonstruktion eine Reihe von Vorteilen, die insbesondere die Fertigung deutlich vereinfachen. Der Apparat besteht dann nicht aus einer großen Anzahl drucktragender Schweißnähte. Auch können die Umlenkbleche unverändert beibehalten werden und müssen keine erhöhten Wandstärken etc. aufweisen. Das vom Hemd umschlossene Rohrbündel kann wie bisher in den drucktragenden Mantel eingeschoben werden. Dabei können die Spaltmaße allerdings nahezu beliebig groß gewählt werden, wodurch auch an dieser Stelle die Montage vereinfacht wird.

Der Einsatz der hier vorgestellten Doppelmantelkonstruktion ermöglicht es, die ansonsten auftretenden Leckageströme zwischen den Umlenkblechen und dem äußeren Mantel zu eliminieren. Der Entfall der Leckageströmung wirkt sich auf mehrere Weisen günstig auf die Wärmeübertragung innerhalb des Reaktors aus. Der wichtigste Effekt ist dabei die Erhöhung des Massenstroms, der den geplanten Weg zwischen den Umlenkblechen hindurch nimmt. Diese Erhöhung des Massenstromes hat eine Vergrößerung der Strömungsgeschwindigkeit zur Folge, die direkt zu einer Verbesserung des Wärmeübergangs zwischen Fluid und Rohr führt. Weiterhin nehmen bei gleicher Wärmeleistung innerhalb eines Segmentes die radialen Temperaturgradienten deutlich ab. Diese Verringerung der Temperaturgradienten entlang eines Segmentes hat wiederum eine höhere mittlere Temperaturdifferenz zwischen Mantelseite und Rohrseite zur Folge, sodass auch durch diese Tatsache von einer Verbesserung der Wärmeübertragung auszugehen ist.

Die Hemdkonstruktion wirkt sich bei MTBE-Spaltung in Verbindung mit dem eingesetzten Rohrreaktor auf zwei Weisen positiv auf die Reaktion aus. Der erste positive Effekt ist die verbesserte Wärmeübertragung in dem Apparat, die zu einem konstanteren Temperaturprofil entlang der Rohrachse führt. Der reaktionsbedingte Temperaturabfall fällt somit geringer aus als bei einem vergleichbaren Apparat ohne eine solche Hemdkonstruktion. Der zweite wichtige Effekt ist die Erhöhung des Massenstroms durch die einzelnen Segmente des Mantelraumes, wodurch das radiale Temperaturprofil verringert wird. Dieser Effekt wirkt sich verbessernd auf die Produktqualitäten aus.

Neben Rohrbündelapparaten können auch Plattenreaktoren für die Durchführung der Spaltungsreaktion eingesetzt werden. Plattenreaktoren sind analog zu Plattenwärmetauschern aufgebaut. Der Abstand der Platten, zwischen denen sich der Katalysator befindet, beträgt dabei bevorzugt 10 - 80 mm.

Bei der Spaltung von MTBE treten Nebenreaktionen auf. Diese sind entweder auf MTBE oder die Spaltprodukte Isobuten und Methanol zurückzuführen.

Standardmäßig tritt bei der MTBE Spaltung die Bildung von Dimethylether (DME) auf. Dabei reagieren zwei Moleküle Methanol zu DME und Wasser. Vorzugsweise wird der erfindungsgemäße Prozess so betrieben, dass die DME-Selektivität der Reaktion zu DME unter 10 % beträgt, bevorzugt unter 4 %. (DME-Selektivität = 2 x [Mole gebildetes DME] / [Mole umgesetztes MTBE]).

Eine weitere Nebenreaktion ist die Bildung von C8-Kohlenwasserstoffen durch Dimerisierung von Isobuten. Diese bestehen hauptsächlich aus einem Gemisch aus 2,4,4-Trimethylpenten-1 und 2,4,4-Trimethylpenten-2.

In der Regel in deutlich geringerem Maße wird die Bildung von weiteren Nebenprodukten beobachtet. Dazu gehören beispielsweise Isobutan, Isopren und Dimethoxymethan sowie höherer Oligomere (C12-KWSt, C16-KWSt).

Zu den Nebenreaktionen kommen meisten noch parallel ablaufende Reaktionen hinzu, bei denen Verunreinigungen aus dem Reaktorzulauf reagieren. Darunter fällt beispielsweise die Spaltung von im MTBE enthaltenen 2-Methoxybutan. Aus diesem können sich durch Abspaltung von Methanol 1-Buten und 2-Butene bilden. Aus im MTBE enthaltenen 3-Methoxy-1-buten oder 1-Methoxy-2-buten kann in der Spaltung 1,3-Butadien gebildet werden.

### Verfahrensschritt d): Isobuten-Abtrennung

Die Spaltprodukte (VI) aus dem Reaktor werden in Schritt d) des erfindungsgemäßen Verfahrens destillativ unter Erhalt einer Fraktion (VII), die jeweils mehr als 50 Massen-% der in den Spaltungsprodukten (VI) enthaltenen Methanol-, TBA- und Wasser-Menge enthält, getrennt.

Tabelle 2 zeigt die Reinstoffsiedepunkte von im Reaktoraustrag typischerweise enthaltenen Komponenten bei 0,5 MPa(abs). Neben Isobuten sind als Leichtsieder weitere C4-Kohlenwasserstoffe (1-Buten, 2-Butene) und DME enthalten. Isopren und Dimethoxymethan sind Beispiele für Mittelsieder mit Siedepunkten, die zwischen MTBE und denen der C4-Kohlenwasserstoffe liegen. Die Mittelsieder werden zum Teil in der Reaktion gebildet oder gelangen als Verunreinigungen über den Zulauf in die Spaltung. Als Hochsieder, also Komponenten mit einem höheren Siedepunkt als MTBE, sind beispielsweise tert.-Butanol, Diisobuten und 2-Methoxybutan enthalten.

Die Angebe für den Wassergehalt bezieht sich in Tabelle 2 auf eine Betriebsweise des Reaktors ohne zusätzliche Zugabe von Wasser in den Strom (V). Wird hier zusätzliches Wasser zugegeben, so erhöht sich typischer Weise der Gehalt an Wasser und an TBA im Reaktoraustrag.

**Tabelle 2: Reinstoffsiedepunkte von im Reaktoraustrag typischerweise enthaltenen Komponenten bei 0,5 MPa_{(abs)}, typische Zusammensetzung**

| *Reinstoff* | *Siedetemp.[°C]* | *Typischer Anteil* |
|---|---|---|
| Dimethyether | 19,2 | 0,1 - 2,5 Massen-% |
| Isobuten | 42,7 | 48 - 65 Massen-% |
| 1-Buten | 43,4 | 10 - 350 Massen-ppm |
| 1,3-Butadien | 45,1 | 0 - 50 Massen-ppm |
| trans-2-Buten | 51,4 | 20 - 750 Massen-ppm |
| cis-2-Buten | 54,1 | (Summe 2-Butene) |
| 2-Butene | 51,4 | 20 - 850 Massen-ppm |
| Isopren | 90,4 | 0 - 100 Massen-ppm |
| Dimethoxymethan | 95,9 | 0 - 150 Massen-ppm |
| Methanol | 111,5 | 27 - 37 Massen-% |
| MTBE | 113,8 | 3 - 25 Massen-% |
| 2-Methoxybutan | 120,8 | 50 - 1700 Massen-ppm |
| tert.-Butanol | 131,3 | 0 - 2500 ppm |
| Wasser | 151,8 | 0,1 - 2 Massen-% |
| Diisobuten | 171,2 | 100 - 1500 Massen-ppm |

In einer bevorzugten Ausführungsform **AFF5** erfolgt die destillative Auftrennung der Spaltprodukte (VI) in genau einer Destillationskolonne. Diese Destillationskolonne weist vorzugsweise von 20 bis 55 theoretische Trennstufen, bevorzugt von 25 bis 45 und besonders bevorzugt von 30 bis 40 theoretische Trennstufen auf. Das Rücklaufverhältnis beträgt, in Abhängigkeit von der realisierten Stufenzahl, der Zusammensetzung des Reaktoraustrags und der erforderlichen Reinheiten von Destillat und Sumpfprodukt, vorzugsweise kleiner 5, bevorzugt kleiner 1. Der Betriebsdruck der Kolonne kann vorzugsweise zwischen 0,1 und 2,0 MPa_{(abs)} eingestellt werden. Dabei ist es vorteilhaft, die Kolonne bei einem niedrigeren Druck als den Druck, mit dem der Spaltungsreaktor betrieben wird, zu betreiben. Dann können die Spaltprodukte (VI) gasförmig oder teilweise gasförmig, nach partieller Kondensation, in die Destillationskolonne überführt werden. Auf einen Verdichter zur Anhebung des Drucks oder eine vollständige Kondensation kann so verzichtet werden.

Besonders bevorzugt werden die Reaktionsprodukte (VI) nach partieller Kondensation in die Destillationskolonne überführt. Dabei werden bevorzugt 30-70 Massen-%, besonders bevorzugt 40 - 70 Massen-% des Gasstroms kondensiert. Der nicht kondensierte Gasstrom wird direkt, der kondensierte falls notwendig nach Druckerhöhung durch eine Pumpe, in die Kolonne eingebracht. Die bei der Abkühlung und partiellen Kondensation freiwerdende Wärme kann dabei zur Wärmeintegration innerhalb des Prozesses oder mit anderen Prozessteilen genutzt werden. Diese Wärmeintegration kann nach bekannten Standards der Technik, beispielsweise über Sumpfverdampfer oder Vorwärmer bzw. Vorverdampfung im Kolonnenzulauf, erfolgen.

Die Einspeisung der Gasphase und der Flüssigphase kann dabei an gleicher oder unterschiedlicher Stelle der Kolonne erfolgen. Bevorzugt erfolgt die Einspeisung der Flüssigphase auf dem gleichen Boden oder ein bis fünf Böden unterhalb der Einspeisung der Gasphase.

Die bei der partiellen Kondensation der Gasphase freiwerdende Energie wird bevorzugt an anderer Stelle des Prozesses genutzt, beispielsweise zur Beheizung einer Kolonne oder zur Vorwärmung des Reaktorzulaufs.

Um Isobuten am Kolonnenkopf gegen Kühlwasser kondensieren zu können, ist ein Druck von ca. 0,5 MPa(abs) notwendig. Wird die Spaltung beispielsweise bei einem Druck von 0,65 MPa(abs) betrieben, kann es vorteilhaft sein, wenn die Destillationskolonne mit einem Betriebsdruck von 0,55 bis 0,6 MPa(abs) durchgeführt wird. Zur Beheizung des Verdampfers der Kolonne kann z. B. 0,4 MPa-Dampf eingesetzt werden.

Als Sumpfprodukt wird die Fraktion (VII) erhalten, die jeweils mehr als 50 Massen-% der in den Spaltungsprodukten (VI) enthaltenen Methanol-, TBA- und Wasser Menge enthält. Bei Aufarbeitung entsprechend dieser Ausführungsform AFF5 enthält die Fraktion (VII) zudem noch MTBE. Bevorzugt weist die nach AFF5 erhaltene Fraktion (VII) jeweils mehr als 90 Massen-% der in den Spaltprodukten (VI) enthaltenen Methanol-Menge, mehr als 98 Massen-% der in den Spaltprodukten (VI) enthaltenen TBA-Menge, mehr als 85 Massen-% der in den Spaltprodukten (VI) enthaltenen Wasser-Menge und mehr als 98 Massen-% der in den Spaltprodukten (VI) enthaltenen MTBE-Menge auf. Besonders bevorzugt enthält die so abgetrennte Fraktion praktisch die Gesamtmenge der in den Spaltprodukten (VI) enthaltenen MTBE-, TBA-, 2-Methoxybutan- und Diisobuten-Menge.

Die im Austrag der Spaltung enthaltenen Mittelsieder werden bevorzugt ebenfalls mit der so erhaltenen Fraktion (VII) abgetrennt, bevorzugt von 60 bis 100 Massen-%, besonders bevorzugt von 80 bis 98 Massen-%.

Die typische Zusammensetzung des Stroms (VII) ergibt sich einfach in Kombination mit den in Tabelle 2 angegebenen Konzentrationsbereichen.

Am Kopf der Kolonne wird die Fraktion (XVII) erhalten, die hauptsächlich Isobuten, Methanol (aufgrund der Azeotropbildung mit C4-Kohlenwasserstoffen) und DME enthält. Als Spuren können 1-Buten, 2-Butene, 1,3-Butadien und Mittelsieder wie Isopren und Dimethoxymethan enthalten sein. Bevorzugt liegt der Gehalt an Mittelsiedern in Summe unter 50 ppm, besonders bevorzugt liegt der Gehalt an Isopren unter 20 ppm, der an Dimethoxymethan unter 30 ppm.

Der Gehalt an Methanol im Destillat beträgt typischer Weise 2 bis 4 Massen-%. Bezogen auf den methanolfrei gerechneten Destillatstrom beträgt die Konzentration an Isopren vorzugsweise kleiner 25 ppm, bevorzugt kleiner 1 ppm, der Gehalt an Dimethoxymethan vorzugsweise kleiner 10 ppm, bevorzugt 1 ppm und der Gehalt an MTBE vorzugsweise kleiner 5 ppm, bevorzugt kleiner 0,1 ppm.

In einer weitern bevorzugten Ausführungsform **AFF6** wird eine erste Destillation analog der in **AFF5** durchgeführt, das Sumpfprodukt der Destillation aber in einer zweiten Destillation weiter aufgereinigt. Dabei wird eine hauptsächlich MTBE- und Methanol-haltige Kopffraktion erhalten. Daneben wird eine im Wesentlichen MTBEfreie, Methanol-reiche Sumpffraktion erhalten, in der auch der größte Teil des TBAs und des Wassers enthalten ist. Die C8-Kohlenwasserstoffe sind bei Betrieb der Kolonne ohne zusätzlichen Seitenabzug ebenfalls Bestandteil der Sumpffraktion. Es ist jedoch möglich, die C8-Kohlenwasserstoffe durch einen Seitenabzug der Kolonne zumindest teilweise auszuschleusen. Ein derartiges Verfahren ist beispielsweise in DE 10 2006 040434 beschrieben. Die Sumpffraktion wird dann als Fraktion (VII) in Schritt e) des erfindungsgemäßen Verfahrens geführt.

Diese zweite Destillationskolonne weist vorzugsweise 20 bis 75 theoretische Trennstufen, bevorzugt 30 bis 65 und besonders bevorzugt 35 bis 50 auf. Es kann vorteilhaft sein, wenn die Kolonne) in Abhängigkeit von der realisierten Stufenzahl und des im Spaltungsreaktor erzielten MTBE-Umsatzes, mit einem Rücklaufverhältnis, von kleiner 10, bevorzugt von 0,5 bis 5 betrieben wird. Der Betriebsdruck der Kolonne wird vorzugsweise auf einen Wert im Bereich von 0,05 bis 1 MPa(abs), bevorzugt von 0,1 bis 0,3 MPa(abs) eingestellt. Zur Beheizung der Kolonne kann z. B. 0,4 MPa(abs) Dampf eingesetzt werden. Die Kondensation kann, je nach gewähltem Betriebsdruck, gegen Kühlsohle, Kühlwasser oder Luft erfolgen.

Das auf diese Weise im Sumpf erhaltene Fraktion (VII) enthält vorzugsweise größer 95, besonders bevorzugt größer 97 Massen-% Methanol. Der TBA-Gehalt im Sumpfprodukt beträgt vorzugsweise zwischen 100 und 1000 Massen-ppm und der Wassergehalt vorzugsweise von 0,3 bis 2,0 Massen-%. Die im Reaktionsteil entstandenen Mittelsieder, z. B. Isopren und Dimethoxymethan, sind vorzugsweise mit kleiner 200 ppm, bevorzugt kleiner 100 ppm und besonders bevorzugt mit kleiner 50 ppm enthalten.

Der hauptsächlich MTBE und Methanol-haltige Kopffraktion enthält neben dem Hauptbestandteil MTBE noch Methanol und die im Reaktionsteil entstandenen Mittelsieder, beispielsweise Isopren und/oder Dimethoxymethan. Dieser Strom wird bevorzugt ganz oder teilweise in Schritt b) des erfindungsgemäßen Verfahrens geführt. Besonders bevorzugt wird er der in Kombination mit der Ausführungsform AFF4 des Verfahrensschrittes in die mittlere der drei Kolonnen geführt, wobei die Mittelsieder in dieser Kolonne als Kopfprodukt mit abgetrennt werden.

### Verfahrensschritt e): Wasser-Abtrennung

Aus der in Verfahrensschritt d) erhaltenen Fraktion (VII) wird in Verfahrensschritt e) unter Erhalt einer Fraktion (VIII) Wasser abgetrennt. Grundsätzlich kann dabei die Abtrennung des Wassers durch alle technisch anwendbaren Verfahren wie Permeation, Pervaporation, Adsorption (beispielsweise Wechseldruckadsorption an Molsieben) oder Destillation vorgenommen werden. Bevorzugt erfolgt die Abtrennung des Wassers durch Destillation in einer oder zwei Destillationskolonnen.

In einer bevorzugten Ausführungsform AFF7 wird das Wasser durch Destillation in einer einzigen Destillationskolonne abgetrennt. Dabei wird das Wasser im Sumpf der Kolonne, alle anderen Komponenten am Kopf der Kolonne erhalten.

Die Destillationskolonne weist vorzugsweise 15 bis 50 theoretische Trennstufen, bevorzugt 15 bis 40 und besonders bevorzugt 20 bis 35 auf. Es kann vorteilhaft sein, wenn die Kolonne in Abhängigkeit von der realisierten Stufenzahl und des im Spaltungsreaktor erzielten MTBE-Umsatzes, mit einem Rücklaufverhältnis, von kleiner 2, bevorzugt von 0,5 bis 1 betrieben wird. Der Betriebsdruck der Kolonne wird vorzugsweise auf einen Wert im Bereich von 0,05 bis 1,0 MPa(abs), bevorzugt von 0,1 bis 0,5 MPa(abs) eingestellt. Zur Beheizung der Kolonne kann z. B. 0,4 MPa Dampf eingesetzt werden. Die Kondensation kann, je nach gewähltem Betriebsdruck, gegen Kühlsohle, Kühlwasser oder Luft erfolgen.

In einer weiteren bevorzugten Ausführungsform AFF8 wird die Abtrennung des Wassers in zwei Destillationskolonnen durchgeführt, die als Zweidruckdestillation betrieben werden. Dabei wird z. B. die erste Kolonne bei einem niedrigerem Druck als die zweite Kolonne betrieben und mit der Kondensationswärme der Kopfbrüden der zweiten Kolonne der Sumpf ersten Kolonne beheizt. Die erste der beiden Destillationskolonne weist dabei vorzugsweise 5 bis 35 theoretische Trennstufen, bevorzugt 10 bis 20 und besonders bevorzugt 15 bis 25 auf. Das Rücklaufverhältnis beträgt 0,1 bis 2, bevorzugt von 0,5 bis 1. Der Betriebsdruck der ersten Kolonne wird vorzugsweise auf einen Wert im Bereich von 0,01 bis 1,0 MPa(abs), bevorzugt von 0,05 bis 0,2 MPa(abs) eingestellt. Die Kondensation kann, je nach gewähltem Betriebsdruck, gegen Kühlsohle, Kühlwasser oder Luft erfolgen. Die zweite der beiden Destillationskolonnen weist vorzugsweise 10 bis 45 theoretische Trennstufen, bevorzugt 18 bis 38 und besonders bevorzugt 20 bis 30 auf. Das Rücklaufverhältnis beträgt 1,0 bis 5,0, bevorzugt von 1,4 bis 2,8. Der Unterschied zwischen dem Sumpfdruck der ersten Kolonne und dem Kopfdruck der zweiten Kolonne bestimmt die treibende Temperaturdifferenz im Verdampfer der ersten Kolonne (= Kondensator der zweiten Kolonne). Der Betriebsdruck der zweiten Kolonne wird vorzugsweise auf einen Wert im Bereich von 0,05 bis 0,5 MPa(abs) oberhalb des Sumpfdruckes der ersten Kolonne eingestellt, bevorzugt von 0,15 bis 0,25 MPa(abs) oberhalb. Zur Beheizung der zweiten Kolonne kann z. B. Dampf eingesetzt werden. Gegebenenfalls ist es zu besseren Regelung des Kolonnensystems sinnvoll, die erste Kolonne mit einen zusätzlichen Verdampfer, der z. B. mit Dampf betrieben wird, und die zweite Kolonne mit einem zusätzlichen Kondensator, der je nach gewähltem Betriebsdruck z. B. mit Kühlsohle, Kühlwasser oder Luft betrieben wird, auszustatten.

Alternativ zur Beheizung der einen bzw. zwei Trocknungskolonnen mit Dampf kann eine Wärmeintegration mit anderen Prozessströmen, beispielsweise den Spaltgasen aus dem Reaktor, erfolgen. Die Wärmeintegrationen erfolgen nach bekanntem Stand der Technik, beispielsweise über Sumpfverdampfer oder Vorwärmer bzw. Vorverdampfung im Kolonnenzulauf.

Bei der Ausführung der Wasserabtrennung als eine Destillationskolonne wird der Destillatstrom, bei Ausführung als zwei Destillationskolonnen die Summe der beiden anfallenden Destillatströme als Fraktion (VIII) erhalten. Hauptbestandteile der Fraktion (VIII) sind Methanol und MTBE, der Wassergehalt in Fraktion (VIII) beträgt weniger als 1 Massen-%, bevorzugt weniger als 0,5 Massen-%, besonders bevorzugt weniger als 0,1 Massen-%. Die erhaltene Fraktion (VIII) wird vollständig oder teilweise in Schritt a) des erfindungsgemäßen Verfahrens zurückgeführt.

Der Sumpfstrom (XLI) der Kolonne bzw. der zweiten Kolonne besteht zum überwiegenden Teil aus Wasser mit Spuren von Methanol und gegebenenfalls von weiteren organischen Komponenten. Vorzugsweise ist der Wassergehalt größer als 99,9 Massen-%, bevorzugt größer als 99,99 Massen-% und besonders bevorzugt größer 99,999 Massen-%. Dieser Strom kann entweder als Prozesswasser genutzt werden, z. B. in einem der im Verfahren vorhandenen Extraktionsschritte, oder als Abwasser, gegebenenfalls nach weiterer Reinigung, ausgeschleust werden.

Bei Einsatz von sauren Katalysatoren in Verfahrensschritt c) kann der Reaktoraustrag (VI) einen sauren pH-Wert aufweisen. Dies kann zur Folge haben, dass in den nachfolgenden Verfahrensschritten d) und e), insbesondere im Sumpfbereich der Kolonne und der beiden Kolonnen in Verfahrensschritt d) und in dem folgenden Verfahrensschritt e), keine preiswerten unlegierten Stähle eingesetzt werden können, sondern zur Vermeidung von Korrosion aufgrund der sauren Bedingungen höherwertige, hochlegierte Stähle eingesetzt werden müssen. Um dies zu vermeiden, kann in Verfahrensschritt d) oder in Verfahrensschritt e) ein Laugestrom zudosiert werden. In Verfahrenssschritt d) erfolgt die Zudosierung vorzugsweise in Strom (VI), bevorzugt in die Kolonne bzw. in die erste Kolonne, unterhalb der Zugabe des Zulaufs. In Verfahrensschritt e) erfolgt die Zudosierung der Lauge vorzugsweise in Strom (VII), bevorzugt in die Kolonne bzw. in die erste Kolonne, unterhalb der Zugabe des Zulaufs.. Die Lauge wird mit Strom (XLI) wieder aus dem Verfahren ausgeschleust. Damit besteht keine Gefahr, dass die die Lauge in Verfahrensschritt a) zurückgeführt wird und hier ggf. zu einer Schädigung des eingesetzten Katalysators führt.

Als Laugen können wässrige Lösungen von Alkalimetallhydroxid oder Erdalkalimetallhydroxid verwendet werden. Bevorzugt werden Natronlauge und Kalilauge, insbesondere Natronlauge eingesetzt. Die Konzentration der Hydroxide in der wässrigen Lösung beträgt 0,5 bis 30 Massen-%, insbesondere 2 bis 5 Massen-%. Bei Natronlauge liegt der Natriumhydroxid-Gehalt vorzugsweise im Bereich von 0,5 bis 30 Massen-%, insbesondere im Bereich von 1 bis 5 Massen-%, ganz besonders im Bereich von 2,5 bis 3,5 Massen-%. Die Lauge wird so dosiert, dass sich im Sumpfstrom XLI bevorzugt ein pH-Wert von größer-gleich 8, bevorzugt von 8 bis 12 einstellt.

### Aufarbeitung zu hochreinem Isobuten

Marktgängige Isobutenqualitäten sind üblicherweise praktisch frei von Methanol. Der bei der destillativen Trennung der Spaltprodukte (VI) anfallende Isobutenstrom enthält noch Methanol. Dies kann nach an sich bekannten Verfahren, beispielsweise durch Extraktion, abgetrennt werden. Die Extraktion von Methanol aus dem Isobuten kann beispielsweise mit Wasser oder einer wässrigen Lösung als Extraktionsmittel z. B. in einer Extraktionskolonne durchgeführt werden.

Vorzugsweise wird die Extraktion mit Wasser oder einer wässrigen Lösung in einer Extraktionskolonne durchgeführt, die vorzugsweise 4 bis 16 theoretische Trennstufen aufweist. Das Extraktionsmittel durchströmt die Extraktionskolonne in Bezug auf den zu extrahierenden Strom vorzugsweise im Gegenstrom. Die Extraktion wird vorzugsweise bei einer Temperatur von 15 bis 50 °C, bevorzugt 25 bis 40 °C durchgeführt. Beispielweise kann bei der Verwendung einer Extraktionskolonne mit mehr als 6 theoretischen Trennstufen, die bei einem Druck von 0,9 MPa(abs) und einer Temperatur von 40 °C betrieben wird, ein wassergesättigtes Isobuten mit einem Methanolgehalt von unter 10 ppm erhalten werden.

Der bei der Extraktion erhaltene methanolhaltige Wasserextrakt kann destillativ in Wasser und Methanol getrennt werden. Das Wasser kann als Extraktionsmittel in die Extraktionsstufe zurückgeführt werden. Das Methanol wird bevorzugt in Schritt a) des erfindungsgemäßen Verfahrens zurückgeführt.

Der feuchte Isobutenstrom aus der Extraktionskolonne kann in einer oder mehreren weiteren Destillationskolonnen durch Abtrennung von Wasser und optional von DME zu trockenem Isobuten aufgearbeitet werden. Das trockene Isobuten wird dabei als Sumpfprodukt erhalten. Im Kondensationssystem am Kopf der Kolonne kann nach einer Phasentrennung Wasser flüssig und DME mit Restmengen Isobuten flüssig und /oder gasförmig abgezogen werden. Eine für die Trocknung bevorzugt eingesetzte Destillationskolonne weist vorzugsweise von 30 bis 80 theoretische Trennstufen, bevorzugt von 40 bis 65 theoretische Trennstufen auf. Das Rücklaufverhältnis beträgt, in Abhängigkeit von der realisierten Stufenzahl und der erforderlichen Reinheit des Isobutens, vorzugsweise kleiner 60, bevorzugt kleiner 40.

Der Betriebsdruck der Kolonne kann vorzugsweise zwischen 0,1 und 2,0 MPa(abs) eingestellt werden. Der am Kopf der Kolonne erhaltene DME-reiche Strom kann, falls nötig, weiter destillativ getrennt werden.

Aus dem Strom VI kann zudem ein Teil des DME optional bereits bei der Destillation in Schritt d) abgetrennt werden, indem der Kondensator an der Destillationskolonne bzw. Reaktivdestillationskolonne als Partialkondensator betrieben wird. In diesem Fall kann die im Kopfprodukt enthaltene C4-Fraktion kondensiert werden und ein Teil des gasförmigen Dimethylethers als Abgasstrom abgezogen werden.

Besonders bevorzugt wird im erfindungsgemäßen Verfahren eine Kolonne zur Abtrennung von DME und Wasser mit einem Dekanter zur Abtrennung von Wasser, der sich im Seitenstrom der Kolonne befindet, verwendet. Durch die Einbindung des Dekanters im Seitenstrom der Kolonne können Isobutenverluste minimiert werden. Ein derartiges Verfahren ist beispielsweise auch in der Anmeldung DE 102 38 370 beschrieben. Der feuchte Isobutenstrom aus der Extraktion wird dabei, ggf. nach Abtrennung von restlichem heterogenem Wasser, beispielsweise durch einen Dekanter oder Koaleszer, in eine Kolonne eingespeist. Am Kopf der Kolonne wird DME und im Sumpf trockenes Isobuten erhalten. Unterhalb oder oberhalb der Edukteinleitungsstelle wird ein Seitenstrom aus der Kolonne flüssig entnommen, der in einen Dekanter geführt wird. Im Dekanter wird die wässrige Phase von der an Wasser verarmten organischen Phase getrennt. Das Wasser wird ausgeschleust, die organische Phase in die Kolonne zurückgeführt. Der Abzug des Stroms zum Seitendekanter erfolgt dabei bevorzugt unterhalb des Kolonnenzulaufs, die Rückführung des Stroms vom Dekanter in die Kolonne unterhalb der Entnahmestelle. Die Kolonne weist dabei vorzugsweise eine Trennstufenzahl von 30 bis 80 theoretische Trennstufen, bevorzugt von 40 bis 65 theoretische Trennstufen auf. Das zu reinigende Isobuten wird vorzugsweise oberhalb der 15 bis 30 Trennstufe, jeweils gezählt von oben, eingespeist. Bevorzugt wird zwei bis fünf Trennstufen oberhalb der Einspeisestelle das gesamte Kondensat dieser Trennstufe abgezogen und in den Dekanter geleitet. Nach Abtrennung des Wassers wird die organische Phase ein bis zwei Trennstufen tiefer in die Kolonne zurückgeführt.

Das Rücklaufverhältnis der Kolonne beträgt, in Abhängigkeit von der realisierten Stufenzahl und der erforderlichen Reinheit des Isobutens, vorzugsweise kleiner 60, bevorzugt kleiner 40. Der Betriebsdruck der Kolonne liegt vorzugsweise zwischen 0,1 und 2,0 MPa(abs), besonders bevorzugt zwischen 1,0 und 1,5 MPa(abs).

Das derart gewonnene Isobuten kann z. B. die in Tabelle 3 aufgeführte Zusammensetzung aufweisen.

**Tabelle 3: Typische Zusammensetzung von marktgängigem Isobuten.**

| Massenanteile [kg/kg] | |
|---|---|
| C3-Kohlenwasserstoffe | < 0,000100 |
| Butane | < 0,001000 |
| Isobuten | > 0,999000 |
| 1-Buten / 2-Butene | < 0,001000 |
| Methanol | < 0,000030 |
| C5-Kohlenwasserstoffe | < 0,000500 |
| Wasser | < 0,000050 |
| Oxyenate | < 0,000010 |

| | |
|---|---|
| Oxygenate: Beispielsweise DME, Dimethoxymethan | |

Die im Isobuten enthaltenen linearen Butene (1-Buten, 2-Butene) sind von diesem technisch sinnvoll nicht abtrennbar. Die Bildung der linearen Butene erfolgt unter anderem aus der Spaltung von 2-Methoxybutan, welches im MTBE enthalten sein kann. Daher kann durch vollständige Abtrennung von 2-Methoxybutan vor der Spaltung die Bildung der linearen Butene vermieden werden. Um die Destillationskosten zu begrenzen, kann es jedoch vorteilhaft sein, eine geringe Konzentration an 2-Methoxybutan zuzulassen. Dies ist insbesondere dann möglich, wenn in Schritt c) des Verfahrens ein Katalysator eingesetzt wird, der MTBE schneller als 2-Methoxybutan zersetzt.

Je nach Reinheitsanforderungen sind aber bei Bedarf auch geringere Konzentrationen der Nebenkomponenten erreichbar.

Bei der Aufarbeitung der abgetrennten C4-Kohlenwasserstoffe aus Schritt a) und bei der Aufreinigung der Fraktion (XVII) durch Extraktion mit Wasser oder wässrigen Lösungen fallen Methanol-Wasser Mischungen an, die bevorzugt durch Destillation in Wasser und Methanol getrennt werden. Bevorzugt werden diese beiden Destillationen gemeinsam als eine Destillation ausgeführt. Fallen im Prozess weitere Wasser-Methanol Ströme an, kann es vorteilhaft sein, diese ebenfalls mit in diese Trennung aufzunehmen. Beispielsweise ist es möglich, die Abtrennung von Wasser entsprechend Schritt e) mit diesen Wasser-Methanol-Destillationen zu kombinieren. Besonders bevorzugt ist dabei die Kombination mit der Kolonne, die bei Ausführung als Zweidruckschaltung bei höherem Druck betrieben wird.

Das mit dem erfindungsgemäßen Verfahren hergestellte Isobuten kann z. B. zur Herstellung von Diisobuten, Isobuten-Oligomeren, Polyisobutylen, Butylkautschuk, t-Butylaromaten, Methacrylsäure, Methylmethacrylat, Methallylchlorid, oder Methallylsulfonaten eingesetzt werden. Insbesondere kann es vorteilhaft sein, sowohl das bei der Spaltung erhaltene Methanol als auch das Isobuten zur Herstellung von Methylmethacrylat einzusetzen. Ein solches Verfahren zur Herstellung von Methylmethacrylat wird beispielsweise in EP 1 254 887 beschrieben, auf welches ausdrücklich verwiesen wird.

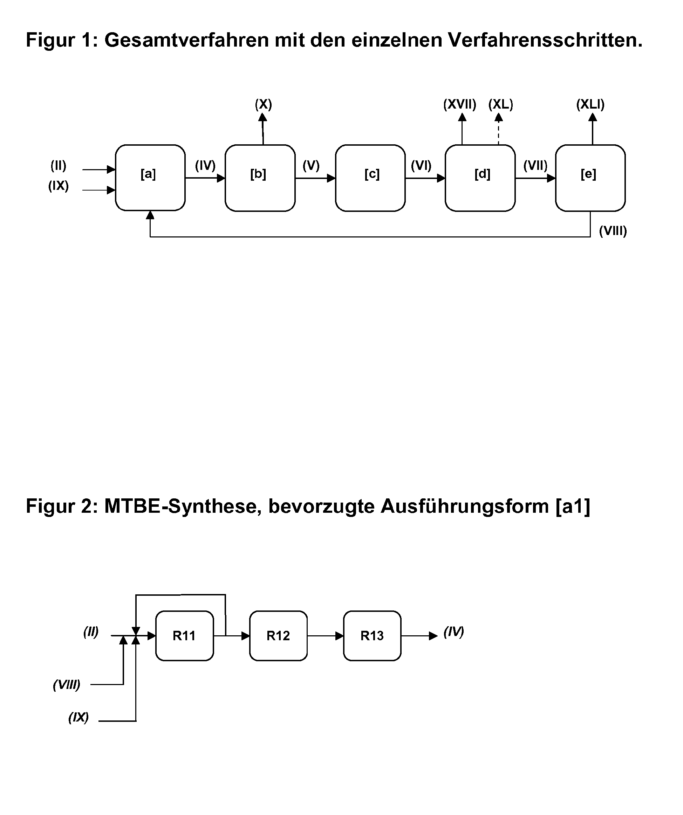

Anhand der Figuren 1 bis 12 wird die Erfindung und einige Ausführungsformen der einzelnen Verfahrensschritte näher erläutert, ohne jedoch die Erfindung auf diese zu beschränken.

Figur 1 veranschaulicht das Verfahren mit seinen einzelnen Verfahrensschritten, die Figuren 2 bis 12 Ausführungsformen der einzelnen Verfahrensschritte.

Figur 1 zeigt die einzelnen Verfahrensschritte mit den dazugehörigen Stoffströmen. Dem Verfahrensschritt [a] werden die Isobuten-haltigen C4-Kohlenwasserstoffe (II), ein Methanol-haltiger Strom (IX) und die Methanol-haltige Fraktion (VIII) zugeführt. In den Strömen (VIII) und (IX) ist das Methanol (III) enthalten, dass mit dem Isobuten (I) aus Strom (II) zur MTBE- und TBA-haltigen Reaktionsmischung (IV) umgesetzt wird.

Der Strom (IX) kann beispielsweise frisches, also nicht im erfindungsgemäßen Prozess zurückgewonnenes, Methanol enthalten oder Methanol, welches bei der Extraktion von Methanol-haltigen C4-Strömen mit Wasser und anschließender Methanol/Wasser-Trennung zurückgewonnen wurde.

Aus der Reaktionsmischung (IV) wird in [b] die MTBE- und TBA-haltige Fraktion (V) abgetrennt. Als zweiter Strom fällt hierbei ein Strom von C4-Kohlenwasserstoffen (X) an, der noch Anteile an Methanol enthält.

Fraktion (V) wird in [c] zu den Spaltprodukten (VI) gespalten, aus der in [d] eine Fraktion (VII) abgetrennt wird, die mehr als 50 Massen-% der in den Spaltprodukten (VI) enthaltenen Methanol, TBA- und Wasser-Menge enthält. Daneben fällt ein Methanol-haltiger Isobutenstrom (XVII) und gegebenenfalls ein MTBE- und Methanol-haltiger Strom (XL) an.

In [e] wird schließlich aus Fraktion (VII) Wasser (XLI) abgetrennt, der verbleibende Strom (VIII) wird in [a] zurück geführt.

In Figur 2 ist eine bevorzugte Ausführungsform des Verfahrensschritts [a] dargestellt. Die Isobuten-haltigen C4-Kohlenwasserstoffe (II) werden zusammen mit den Methanol-haltigen Strömen (VIII) und (IX) gemischt und in einen ersten Reaktor (R11) gefahren. Der Reaktor (R11) wird als adiabatischer Festbettreaktor mit Rückführung betrieben, die beiden folgenden Reaktoren (R12, R13) im geraden Durchgang. Vor den Reaktoren R12 / R13 wird jeweils die Temperatur separat eingeregelt. Aus Reaktor (R13) wird die Reaktionsmischung (IV) erhalten.

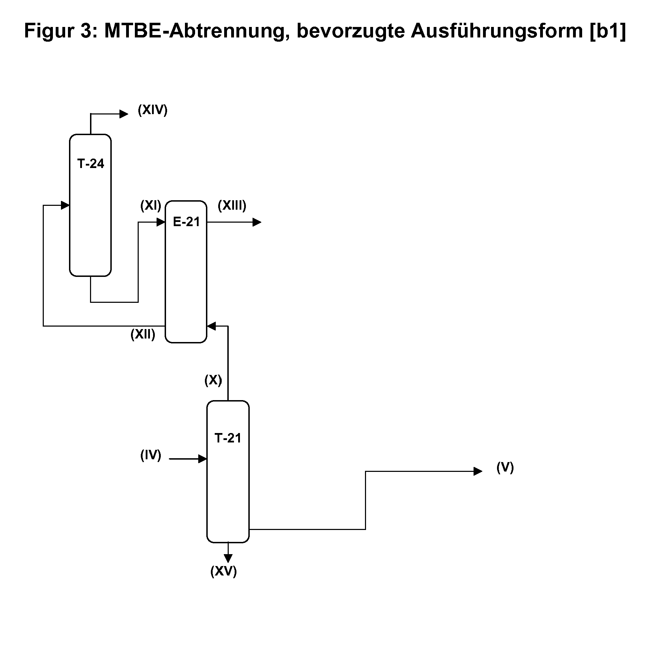

Figur 3 zeigt eine Ausführungsform [b1] des Verfahrensschritts [b]. Die Reaktionsmischung (IV) wird in der Kolonne T-21 in die MTBE- und TBA-haltige Fraktion (V), die als Seitenabzug abgenommen wird, eine Methanol-haltige Kopffraktion von C4-Kohlenwasserstoffen (X) und eine C8-angereicherte Sumpffraktion (XV) getrennt. Diese Ausführung der Kolonne T-21 entspricht der zuvor beschriebenen Ausführungsform AFF1.

Zusätzlich dargestellt ist eine bevorzugte Aufarbeitung der Kopffraktion (X). Aus den Methanol-haltigen C4-Kohlenwasserstoffen (X) wird das Methanol in einer Extraktionskolonne (E-21) im Gegenstrom mit Wasser (XI) das Methanol herausgewaschen. Die Wasser / Methanolmischung (XII) wird in Kolonne T-24 destillativ in das Sumpfprodukt Wasser (XI), das in die Extraktion zurückgeführt wird, und das Kopfprodukt Methanol (XIV) getrennt. Der Strom (XIV) wird bevorzugt als Teilstrom des Stroms (IX) in Schritt [a] eingesetzt.

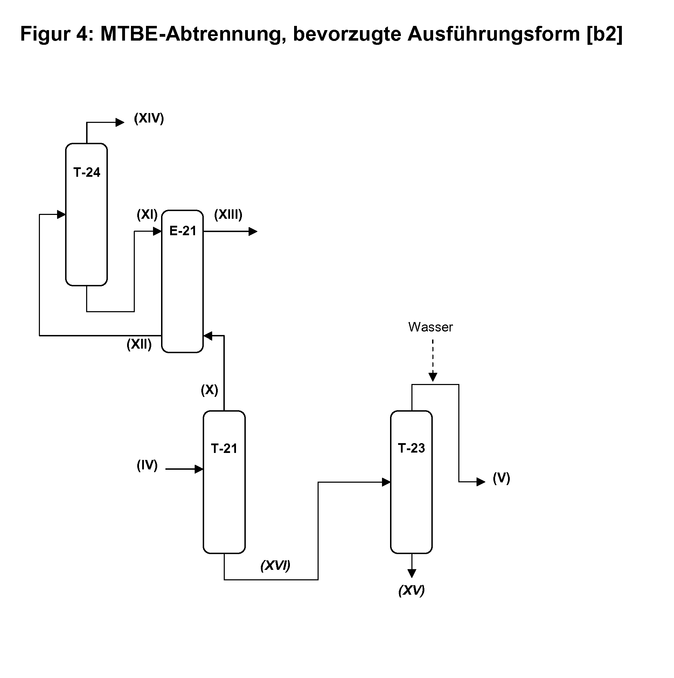

Figur 4 zeigt eine Ausführungsform [b2] des Verfahrensschritts [b]. Die Reaktionsmischung (IV) wird in der Kolonne T-21 in eine MTBE-, 2-Methoxybutan-, C8- und TBA-haltige Sumpffraktion (XVI) und eine Methanol-haltige Kopffraktion von C4-Kohlenwasserstoffen (X) und getrennt.

Die Kolonne T-21 wird in dieser Ausführungsform bevorzugt als Reaktivdestillationskolonne betrieben. Die Ausführung der Kolonne T-21 als Reaktivdestillationskolonne entspricht der zuvor beschriebenen Ausführungsform AFF2.

Die Sumpffraktion (XVI) wird in einer weiteren Destillationskolonne T-23 weiter aufgetrennt. Als Kopfprodukt erhält man die MTBE- und TBA-haltige Fraktion (V), als Sumpfprodukt (XV) werden C8-Kohlenwasserstoffe und 2-Methoxybutan zumindest teilweise abgetrennt. Aufgrund der schwierigen Trennaufgabe MTBE/2-Methoxybutan ist in der Regel auch noch MTBE enthalten. Bevorzugt wird die Kolonne T-23 bei so hohem Druck betrieben, dass die Fraktion (V) am Kopf der Kolonne gasförmig abgezogen und gegebenenfalls nach weiterer Aufheizung direkt in die Spaltreaktion geleitet werden kann. Die Aufreinigung des MTBEs in der Kolonne T-23 entspricht der zuvor beschriebenen Ausführungsform AFF3. Angedeutet ist hier zudem die Option, zum Strom (V) zusätzlich Wasser hinzuzufügen, um den Katalysator zu moderieren. Die Aufarbeitung der Kopffraktion (X) erfolgt analog zur Ausführungsform [b1].

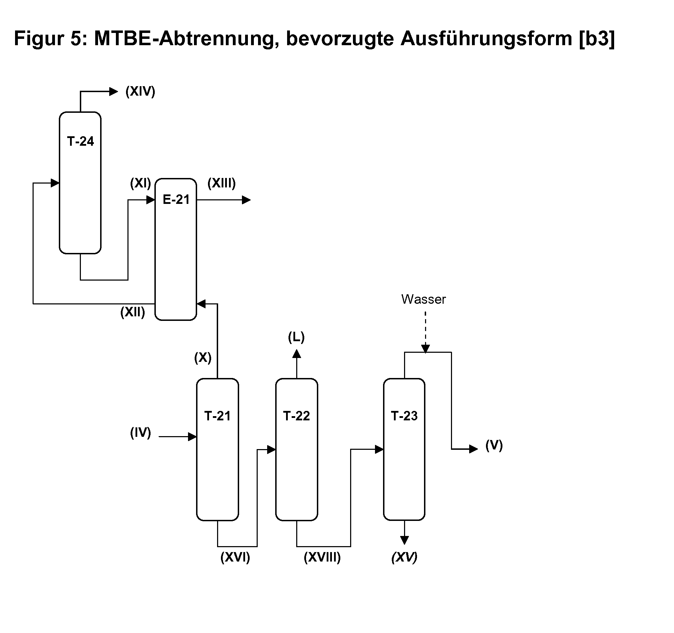

Figur 5 zeigt eine Ausführungsform [b3] des Verfahrensschritts [b]. Die Reaktionsmischung (IV) wird in der Kolonne T-21 in eine MTBE-, 2-Methoxybutan-, C8- und TBA-haltige Sumpffraktion (XVI) und eine Methanol-haltige Kopffraktion von C4-Kohlenwasserstoffen (X) getrennt. Die Kolonne T-21 wird in dieser Ausführungsform bevorzugt als Reaktivdestillationskolonne betrieben. Die Ausführung der Kolonne T-21 als Reaktivdestillationskolonne entspricht wieder der zuvor beschriebenen Ausführungsform AFF2.

Aus der Fraktion (XVI) werden in Kolonne T-22 über Kopf Leicht- und Mittelsieder (L), beispielsweise Restmengen an C4-Kohlenwasserstoffen und C5-Kohlenwasserstoffe, abgetrennt. So können beispielsweise alle Komponenten mit einem Siedepunkt unter 50 °C bei 0,1 MPa(abs) über das Destillat abgetrennt werden. Die Aufreinigung des MTBEs in der Kolonne T-22 entspricht der zuvor beschriebenen Ausführungsform AFF4. Das Sumpfprodukt (XVIII) wird dann in der Kolonne T-23 in die am Kopf erhaltene MTBE- und TBA-haltige Fraktion (V) und das Sumpfprodukt (XV) getrennt, welches eine Zusammensetzung analog zu der in [b2] ausweist. Die Aufreinigung des MTBEs in der Kolonne T-23 entspricht wieder der zuvor beschriebenen Ausführungsform AFF3. Auch ist hier zudem die Option angedeutet, zum Strom (V) zusätzlich Wasser hinzuzufügen, um den Katalysator zu moderieren.

Die Aufarbeitung der Kopffraktion (X) erfolgt analog zur Ausführungsform [b1].

Der Vorteil dieser Ausführungsform [b3] liegt darin, dass in (XVI) enthaltene Reste an C4-Kohlenwasserstoffen und auch mittel siedende Komponenten auf einfache Weise abgetrennt werden können. So gelangen sie weder in den MTBE-Spaltreaktor noch in die Kopffraktion (X).

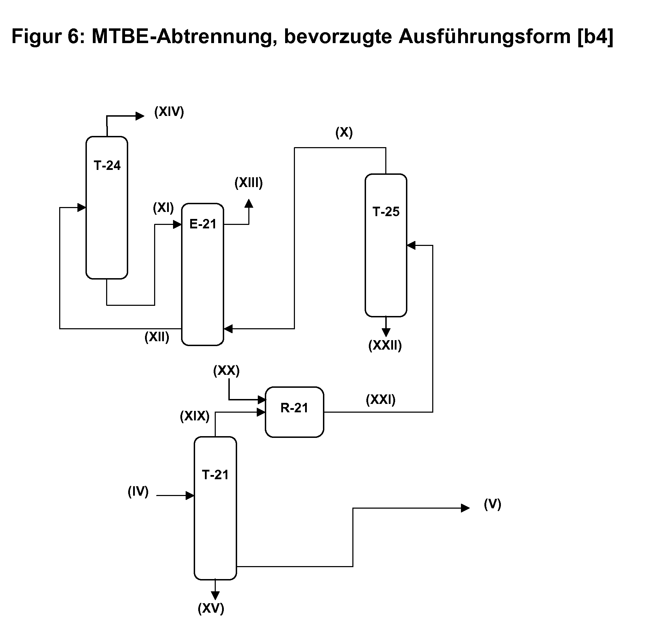

Figur 6 zeigt eine Ausführungsform [b4] des Verfahrensschritts [b]. Die Reaktionsmischung (IV) wird in der Kolonne T-21 in die MTBE- und TBA-haltige Fraktion (V), die als Seitenabzug abgenommen wird und eine C8-angereicherte Sumpffraktion (XV) getrennt. Als Kopffraktion (XIX) wird ein Gemisch aus C4-Kohlenwasserstoffen, Methanol und gegebenenfalls MTBE erhalten. Diese Mischung wird in einem Reaktor R-21 zugeführt, in dem Restmengen an nicht umgesetzten Isobuten weiter zu MTBE abreagieren. Dem Reaktor kann zusätzlich ein Methanol-haltiger Strom (XX) zugeführt werden. Der Reaktoraustrag (XXI) wird anschließend in einer Kolonne T-25 in eine Methanol-haltige C4-Fraktion (X) und eine MTBE-haltige Sumpffraktion (XXII) getrennt. Diese Fraktion wir separat verwertet oder kann vollständig oder teilweise in Schritt [a] des Verfahrens zurückgeführt werden. Die Aufarbeitung der Kopffraktion (X) erfolgt wiederum analog zur Ausführungsform [b1]. In dieser Ausführungsform kann es vorteilhaft sein, die Kolonne T-25 als Reaktivdestillation auszuführen.

Der Vorteil dieser Ausführungsform liegt darin, dass der Isobutenumsatz in Schritt [a] des Verfahrens kleiner sein kann, wodurch sich auch weniger 2-Methoxybutan bildet. Dadurch kann gegebenenfalls auf eine Kolonne T-23, wie sie in den Ausführungsformen [b2] und [b3] verwendet wird, vollständig verzichtet oder die Kolonne deutlich kleiner dimensioniert werden.

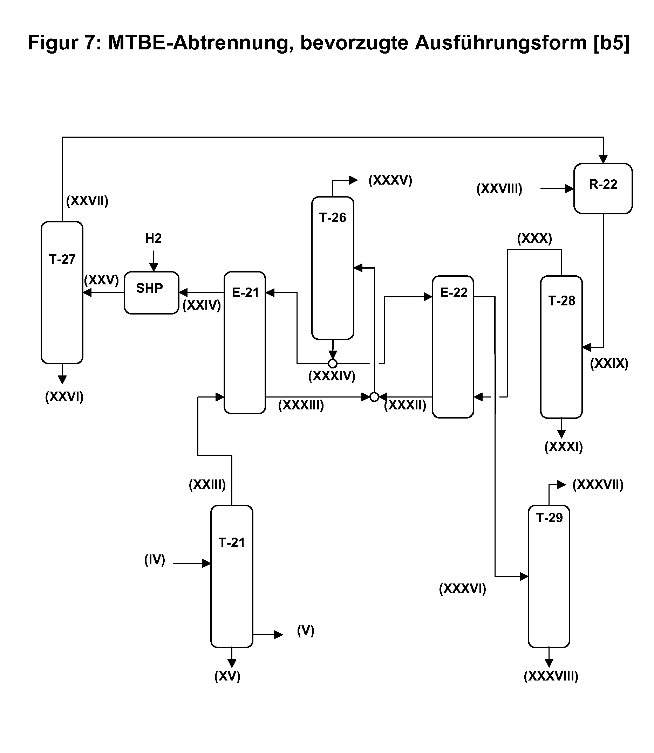

Figur 7 zeigt eine Ausführungsform [b5] des Verfahrensschritts [b]. Hier erfolgt eine Aufreinigung bis zu hochreinem 1-Buten, mit der Besonderheit, dass in der 1-Buten-Aufreinigung noch Restmengen an Isobuten zu MTBE umgesetzt werden.

Die Reaktionsmischung (IV) wird in der Kolonne T-21 in die MTBE- und TBA-haltige Fraktion (V), die als Seitenabzug abgenommen wird und eine C8-angereicherte Sumpffraktion (XV) getrennt. Als Kopffraktion (XXIII) wird eine Methanol-haltige C4-Fraktion (XXIII) abgenommen, die noch Restmengen an Isobuten enthält. Das Methanol wird aus dem Strom (XXIII) mit Wasser (XXXIV) in einer Extraktionskolonne E-21 ausgewaschen. Noch im resultierenden Strom (XXIV) enthaltene Mengen an 1,3-Butadien werden in einer selektiven Hydrierung SHP mit Wasserstoff zu n-Butenen umgesetzt, der Austrag der Hydrierung (XXV) in die Kolonne T-27 geführt. In Kolonne T-27 erfolgt die erste Trennung der C4-Kohlenwasserstoffe. Als Kopfprodukt (XXVII) werden 1-Buten, die Reste an Isobuten, Isobutan und enthaltene Leichtsieder, vor allem DME aus der MTBE-Synthese, erhalten. n-Butan, 2-Butene und gegebenenfalls Restmengen an 1-Buten werden am Sumpf der Kolonne als Strom (XXVI) abgezogen. Gegebenenfalls heterogen im Kopfstrom (XXVII) anfallendes Wasser wird abgetrennt. Das Destillat wird einer Reaktionsstufe R-22 zugeführt, in der das Isobuten mit Methanol (XXVIII) umgesetzt wird. Katalysator und Reaktionsbedingungen entsprechen dabei denen des Verfahrensschrittes a) des erfindungsgemäßen Verfahrens.

Das aus der Reaktion erhaltene Gemisch (XXIX) wird in der Kolonne T-28 in Methanol-haltige C4-Kohlenwasserstoffe (XXX) und eine MTBE-haltige Sumpffraktion (XXXI) getrennt. Die MTBE-Fraktion enthält bevorzugt noch Methanol und wird als Teilstrom von (IX) in Schritt a) des erfindungsgemäßen Verfahrens zurückgefahren.

Aus dem Strom (XXX) wird in der Extraktion E-22 Methanol mit Wasser (XXXIV) ausgewaschen. Die Wasser / Methanol-Ströme (XXXIII) und (XXXII) aus den beiden Extraktionen werden in einer gemeinsamen Kolonne T-26 in Wasser (XXXIV) und Methanol (XXXV) getrennt. Der Strom (XXXV) wird bevorzugt als Teilstrom von (IX) in Schritt a) des erfindungsgemäßen Verfahrens zurückgefahren.

Der Wasser-gesättigte Strom (XXXVI) wird in einer nachfolgenden Destillation T-29 zu hochreinem 1-Buten (XXXVIII) aufdestilliert. Er fällt als Sumpfprodukt der Kolonne an. Am Kopf der Kolonne werden Isobutan und Leichtsieder wie DME erhalten (XXXVII). Zur Abtrennung des Wassers ist es sinnvoll, die Kolonne T-29 mit einem Kopfdekanter auszustatten in dem sich das Wasser als zweite Phase abscheidet und ausgeschleust werden kann.

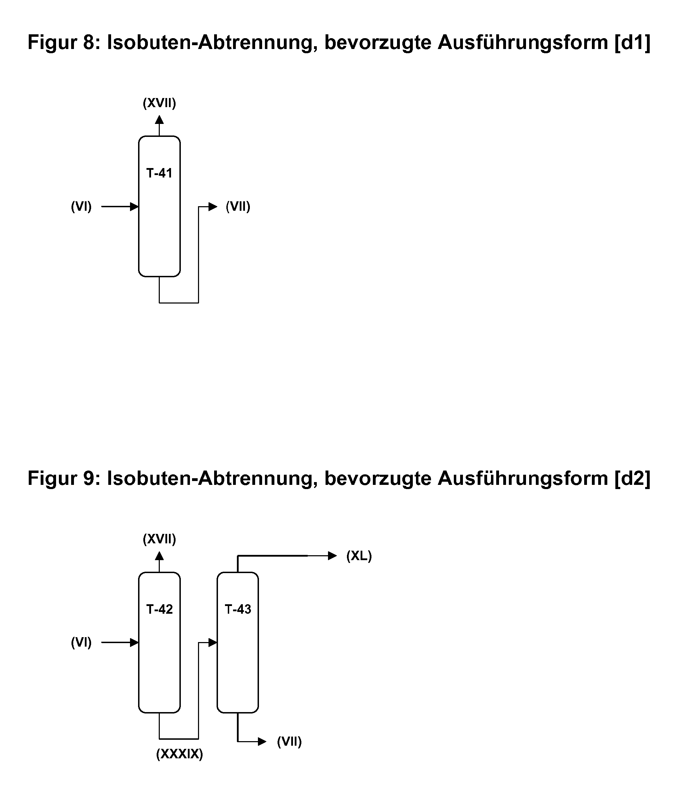

Figur 8 zeigt eine Ausführungsform [d1] des Verfahrensschritts [d]. Die Auftrennung der bevorzugt durch Wärmeintegration mit anderen Prozessströmen teilkondensierten Spaltprodukte (VI) erfolgt in einer einzigen Destillationskolonne T-41, wobei im Sumpf die Fraktion (VII) erhalten wird, die als Hauptbestandteile Methanol und nicht umgesetzten MTBE aufweist. Das Kopfprodukt (XVII) besteht aus Isobuten, Methanol und Leichtsiedern wie DME. Mittelsieder wie C5-Kohlenwasserstoffe, Isopren und Dimethoxymethan werden bevorzugt mit dem Sumpfprodukt erhalten. Die Isobuten-Abtrennung in der Kolonne T-41 entspricht der zuvor beschriebenen Ausführungsform AFF5.

Figur 9 zeigt eine Ausführungsform [d2] des Verfahrensschritts [d]. Die Auftrennung der Spaltprodukte (VI) erfolgt hier in zwei Destillationsschritten. Dies entspricht der zuvor beschriebenen Ausführungsform AFF 6. Das Kopfprodukt der Kolonne T-42 (XVII) besteht aus Isobuten, Methanol und Leichtsiedern wie DME. Mittelsieder wie C5-Kohlenwasserstoffe, Isopren und Dimethoxymethan werden bevorzugt mit dem Sumpfprodukt erhalten. Der Sumpfstrom der Kolonne T-42 (XXXIX) wird in eine zweite Kolonne T-43 geführt. Am Kopf der Kolonne wird ein Azeotrop aus Methanol und MTBE (XL) abgenommen. Als Sumpfstrom die Fraktion (VII) erhalten, die Methanol, TBA und Wasser enthält, aber praktisch frei ist von MTBE.

Das MTBE / Methanol-Azeotrop (XL) kann vollständig oder teilweise in den Prozess zurückgeführt werden. Bevorzugt ist eine Rückführung in den Schritt a) oder b) des Verfahrens. Besonders bevorzugt ist eine Rückführung in eine der Kolonnen T-21, T-22 oder T-23 der bevorzugten Ausführungsformen [b1], [b2], [b3] oder [b4], oder eine Rückführung in den Reaktor R-21 der Ausführungsform [b4].

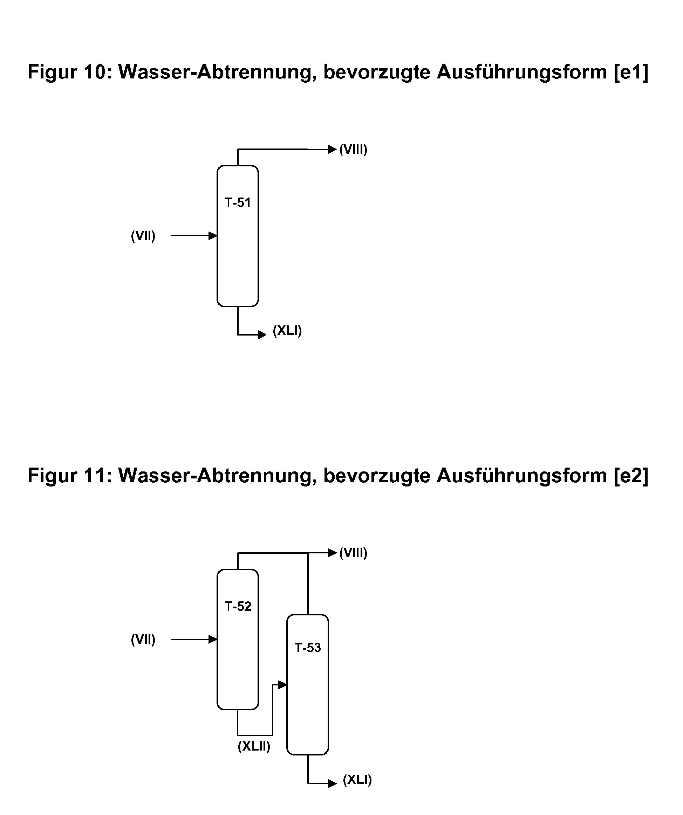

Figur 10 zeigt eine Ausführungsform [e1] des Verfahrensschritts [e]. Die Abtrennung von Wasser aus der Fraktion (VII) erfolgt in nur einer Destillationskolonne T-51. Dabei wird Wasser als Sumpfprodukt (XLI) und die an Wasser verarmte Fraktion (VIII) als Kopfprodukt erhalten. Die Wasser-Abtrennung in der Kolonne T-51 entspricht der zuvor beschriebenen Ausführungsform AFF7.

Figur 11 zeigt eine Ausführungsform [e2] des Verfahrensschritts [e]. Die Abtrennung von Wasser aus der Fraktion (VII) erfolgt in zwei Destillationskolonnen, die in einer Zweidruckschaltung betrieben werden. Dies entspricht der zuvor beschriebenen Ausführungsform AFF 8. In der ersten Kolonne T-52, die unter geringerem Druck betrieben wird, wird als Kopfprodukt der erste Teilstrom der Fraktion (VIII) erhalten. Der Sumpfstrom (XLII), der hauptsächlich Methanol und Wasser enthält, wird in einer zweiten Kolonne, die unter höherem Druck betrieben wird, weiter aufgetrennt. Dabei wird am Kopf der zweite Teilstrom der Fraktion (VIII) erhalten, im Sumpf fällt Wasser (XLI) an. Die Beheizung der Kolonne T-52 erfolgt bevorzugt mit den Brüden der Kolonne T-53.

Die Ausführung des Verfahrensschrittes e) in Form einer Zweidruckschaltung von zwei Kolonnen bietet den Vorteil, dass sie in der Regel deutlich weniger Energie benötigt als die Trennung in nur einer Kolonne. Besonders bevorzugt ist eine Variante, in der der Zulauf mindestens einer der Kolonnen unter Ausnutzung eines Teils der Kondensationswärme des Spaltgases aus Schritt c) weitgehend verdampft wird.

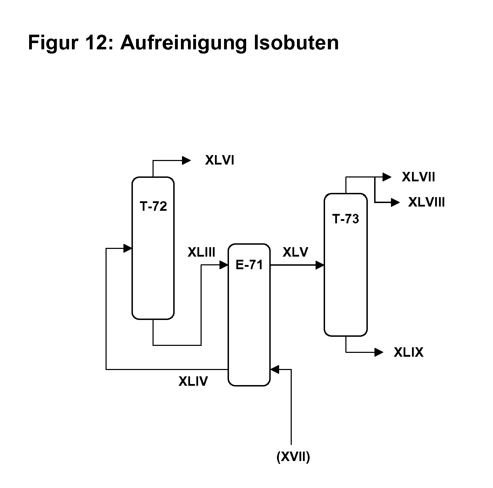

Figur 12 zeigt eine typische Ausführungsform für die weitere Aufarbeitung des in Schritt [d] erhaltenen Methanol-haltigen Isobutenstroms (XVII).
Aus dem Strom (XVII) wird in einer Extraktion E-71 Methanol mit Wasser (XLIII) ausgewaschen. Das Methanol-beladene Wasser (XLIV) wird in der Kolonne T-72 in einen Methanol-haltigen Strom (XLVI) und Wasser (XLIII) getrennt, das Wasser zurück in die Extraktion geführt. Der Strom (XLVI) wird bevorzugt als Teilstrom von (IX) in Schritt a) des erfindungsgemäßen Verfahrens zurückgefahren.
Der Wasser-gesättigte Isobutenstrom (XLV), der noch DME enthält, wird anschließend in der Kolonne T-73 weiter aufgereinigt. Am Kopf der Kolonne wird ein DME-haltiger Strom (XLVII) erhalten, der normalerweise noch Isobuten enthält. Zur Abtrennung des Wassers ist es sinnvoll, die Kolonne T-29 mit einem Kopfdekanter auszustatten in dem sich das Wasser als zweite Phase abscheidet und ausgeschleust werden kann. Im Sumpf der Kolonne wird Isobuten (XLIX) erhalten.

Da der Kopfstrom der Kolonne T-73 oft noch erhebliche Mengen an Isobuten enthält, kann es sinnvoll sein, ihn in einer weiteren Kolonne aufzureinigen (nicht abgebildet). In dieser Kolonne wird am Kopf ein Isobuten-abgereicherter DME-Strom erhalten, im Sumpf ein Isobuten-angereicherter Strom. Dieser Sumpfstrom wird bevorzugt in die Kolonne T-73 zurückgeführt.

Das im erfindungsgemäßen Verfahren für die MTBE-Synthese und Spaltung gemachte Vorgehen lässt sich auch auf den Einsatz anderer Alkohole übertragen. Dies betrifft vor allem die Synthese und Spaltung von Ethyl-tert.-butylether (Einsatz von Ethanol als Alkohol), n-Propyl-tert.-butylether (Einsatz von n-Propanol als Alkohol), n-Butyl-tert.-butylether (Einsatz von n-Butanol als Alkohol) und iso-Butyl-tert.-butylether (Einsatz von iso-Butanol als Alkohol). Bei all diesen Alkoholen kommt zudem hinzu, dass aus ihnen in der Spaltung durch Abspaltung von Wasser Olefine gebildet werden können, wodurch ein zusätzlicher Eintrag von Wasser in den Prozess erfolgt.

### Beispiele

Die folgenden Beispiele sollen die Erfindung erläutern.

### Beispiel 1:

Einer Mischung aus synthetisch hergestelltem Raffinat I und Methanol wurden unterschiedliche Wassermengen zudosiert. Die Mischung wurde dann in einem Laborreaktor eingeleitet. Bei dem Reaktor handelte es sich um einen Rohrreaktor (Länge 1000 mm, Innendurchmesser 21 mm) mit Kühlmantel, durch den ein Wärmeträgeröl (Marlotherm SH der Sasol Olefins & Surfactans GmbH) floss.

Als Katalysator wurde ein saurer lonenaustauscher - Amberlyst® 15 (Rohm & Haas) - verwendet. Der verwendete Katalysator bestand aus Pellets mit einem mittleren Durchmesser von 0,6 - 0,85 mm, mit einer Oberfläche von 45 m2/g und einem Schüttgewicht von 770 g/l. Die Säurekapazität des Katalysators, bezogen auf den feuchten Katalysator, betrug 1,7 eq/ml.

Die Eduktmischung wurde vor Eintritt in den Reaktor in einem Wärmetauscher auf 36 °C vorgewärmt, die Manteltemperatur wurde in allen Versuchen ebenfalls auf 38 °C eingestellt (Temperatur des Marlotherms im Zulauf des Reaktormantels). Der Druck wurde über eine Druckhaltung am Ende des Reaktors auf konstant 1,2 MPa(abs) eingestellt. Der Zulauf wurde auf 510 g/h eingeregelt, was bei einer Katalysatormenge von 273,5 ml einen LHSV-Wert von 3,12 h-1 entspricht. Das in den Reaktor eintretende Eduktgemisch und das den Reaktor verlassende Produktgemisch wurden gaschromatographisch analysiert.

**Tabelle 4: Analysen Reaktorzulauf, Reaktoraustrag und Isobuten-Umsatz für Beispiel 1.**

| | | **Versuch 1** | | **Versuch 2** | | **Versuch 3** | |
|---|---|---|---|---|---|---|---|
| | | **Edukt** | **Produkt** | **Edukt** | **Produkt** | **Edukt** | **Produkt** |
| **Isobuten** | **[M%]** | 13,85 | 0,95 | 14,31 | 2,36 | 13,77 | 9,13 |
| **Methanol** | **[M%]** | 13,57 | 6,25 | 13,61 | 7,08 | 13,23 | 11,47 |
| **MTBE** | **[M%]** | 0,00 | 20,04 | 0,00 | 17,75 | 0,00 | 5,66 |
| **TBA** | **[M%]** | 0,00 | 0,02 | 0,00 | 0,26 | 0,00 | 1,40 |
| **Butane** | **[M%]** | 12,23 | 12,15 | 12,30 | 12,32 | 12,21 | 12,20 |
| **1-Buten** | **[M%]** | 36,56 | 36,53 | 36,20 | 36,59 | 37,14 | 36,93 |
| **2-Butene** | **[M%]** | 23,17 | 23,37 | 22,92 | 23,10 | 22,39 | 22,35 |
| **Wasser** | **[M%]** | **0,02** | **0,01** | **0,11** | **0,05** | **0,65** | **0,28** |
| **Rest** | **[M%]** | 0,60 | 0,67 | 0,55 | 0,49 | 0,61 | 0,58 |
| **Isobuten Umsatz** | **[%]** | | **93,12** | | **83,50** | | **33,69** |

In Tabelle 4 sind die Analysen für Reaktorzulauf, Reaktoraustrag sowie der errechnete Isobuten-Umsatz aufgeführt. In Versuch 1 wurde der Mischung aus synthetisch hergestelltes Raffinat I und Methanol kein zusätzliches Wasser zugemischt, der Wassergehalt von ca. 200 ppm ergibt sich aus dem ursprünglich in Methanol und im Raffinat enthaltenen Wasser.

Zu erkennen ist, dass mit steigendem Wassergehalt der Isobuten-Umsatz deutlich zurückgeht, in Versuch 1 beträgt der Umsatz ca. 93,1 %, in Versuch 2 bei ca. 1100 ppm Wasser im Edukt nur noch 83,5 % und schließlich in Versuch 3 bei ca. 6500 ppm Wasser im Reaktorzulauf nur noch ca. 33,7 %. Gleichzeitig steigt die TBA-Bildung mit steigendem Wassergehalt, bei Versuch 3 wurde im Reaktoraustrag schon über ein Massen-% TBA gemessen.

### Erläuterungen zu den Beispielen 2 und 3:

In den nachfolgenden Beispielen 2 und 3 wurden Rechnungen mit dem stationären Simulationsprogramm ASPEN Plus (Version 2006.5 der Firma AspenTech) durchgeführt, die die Auswirkungen der Wasserausschleusung auf den Gesamtprozess wiedergeben.

Um transparente, reproduzierbare Daten zu erzeugen, wurden nur allgemein zugängliche Stoffdaten eingesetzt. Durch diese Vereinfachung ist es dem Fachmann leicht möglich, die Berechnungen nachzuvollziehen. In den Beispielen wurde die Property-Methode "UNIFAC-DMD" (s. J. Gmehling, J. Li, and M. Schiller, Ind. Eng. Chem. Res. 32, (1993), pp. 178 - 193) benutzt.

Für die Modellierung der Reaktoren R11, R12 und R13, der Reaktivdestillation in der Kolonne T21 in der MTBE-Synthese sowie des Spaltungsreaktors in der MTBE-Spaltung wurden in den Rechnungen kinetische Reaktormodelle verwendet, die auf umfangreichen experimentellen Messdaten mit den jeweiligen Katalysatoren beruhen. In den Beispielen werden daher jeweils auch die Reaktionstemperaturen genannt, die bei der Reaktormodellierung angenommen wurden. Da auch jeweils die Zusammensetzung der ein- und ausgehenden Ströme der Reaktionsstufen genannt werden, ist es dem Fachmann durch Nachstellung der Reaktoren mit fest vorgegebenen Umsätzen möglich, das Beispiel nachzurechnen, ohne die genauen Gleichungen für die Kinetik zu kennen.

### Beispiel 2: (erfindungsgemäß)

Das Beispiel 2 entspricht dem in Figur 1 dargestellten Verfahren, wobei für die Umsetzung des Isobutens eine Variante [a1] gemäß Figur 2, für die MTBE-Destillation eine Variante [b3] gemäß Figur 5, für die Aufreinigung der Spaltprodukte eine Variante [d1] gemäß Figur 8 und schließlich für die Trocknung eine Variante [e1] gemäß Figur 10 angenommen wird.

Als Zulauf zur Anlage wird gemäß Figur 1 bzw. 2 ein C4-Kohlenwasserstoffstrom (II) von 10000 kg/h mit der in Tabelle 5 aufgeführten Zusammensetzung angenommen (typisches Raffinat I, vergleiche mit Tabelle 1). Die Zusammensetzungen des zugeführten Frischmethanols sowie des aus Verfahrenschritt [e] kommenden des Rückführstroms (VIII) sind ebenfalls in Tabelle 5 dargestellt. Die Frischmethanolmenge wurde so eingestellt, dass sich ein molares Verhältnis von Methanol zu Isobuten im Zulauf zum ersten Reaktor von 1,14 ergibt.

**Tabelle 5: Zusammensetzung des Eintrittsstroms (II), des Frischmethanols(IX) und des Rückführstroms (VIII) für Beispiel 2.**

| | Raffinat I (II) | Frischmethanol (IX) | Rückführstrom (VIII) |
|---|---|---|---|
| Massenstrom [kg/h] | 10000,0 | 502,8 | 2109,4 |
| Massenanteile [kg/kg] | | | |
| Dimethylether | | | 0,000025 |
| Isobuan | 0,025000 | | |
| Isobuten | 0,350000 | | 0,000203 |
| 1-Buten | 0,340000 | | |
| 1,3-Butadien | 0,001500 | | |
| n-Butan | 0,080000 | | |
| 2-Butene | 0,202850 | | 0,000001 |
| C5-Kohlenwasserstoffe | 0,000150 | | 0,001300 |
| MTBE | | | 0,147283 |
| 2-Methoxybutan | | | 0,004743 |
| Methanol | | 0,997500 | 0,841886 |
| Tert.-Butanol | | | 0,001342 |
| Wasser | 0,000500 | 0,000250 | 0,000800 |
| Diisobuten | | | 0,002416 |

Der C4-Kohlenwasserstoffstrom (II), das Frischmethanol (IX) und der methanolhaltige Rückführungsstrom (VIII) werden vermischt und in Variante [a1] gemäß Figur 2 den Reaktoren R-11, R-12 und R-13 zugeführt.

Die Umsetzung des in Strom (II) enthaltenen Isobutens erfolgt in drei in Reihe geschalteten, adiabatischen Festbettreaktoren, wobei der erste Reaktor als Kreislaufreaktor ausgeführt ist, wie in Figur 2 dargestellt. Es wird eine Füllung der Reaktoren mit Amberlyst® 15 (Rohm & Haas) angenommen. Der Reaktor R-11 wird mit einem Volumen von 18 m³ modelliert, R-12 mit 10 m³ und R-13 mit 5 m³. Die Eintrittstemperatur in dem Reaktor R-11 beträgt 40 °C, der angenommene Kreislaufstrom 28300 kg/h. Die Eintrittstemperatur in den Reaktor R-12 beträgt 40 °C, die Eintrittstemperatur in den Reaktor R-13 35 °C. Unter diesen Bedingungen ergibt sich ein Umsatz von Isobuten über alle drei Reaktoren von ca. 97,8 %. Als Nebenreaktion werden im verwendeten kinetischen Modell die Bildung von TBA aus Isobuten und Wasser, die Dimerisierung von Isobuten zu Diisobuten, die Reaktion von Methanol zu DME und Wasser sowie die Bildung von 2-Methoxybutan aus n-Butenen berücksichtigt. Damit ergibt sich für den Reaktoraustrag (IV) die in Tabelle 6 aufgeführte Zusammensetzung (Zulauf zur Kolonne T-21).

**Tabelle 6: Zusammensetzung des Zulaufstroms (IV), des Destillats und des Sumpfproduktes (XVI) der Kolonne T-21 für Beispiel 2.**

| | Zulauf T-21 (IV) | Destillat T-21 (X) | Sumpfprod. T-21 (XVI) |
|---|---|---|---|
| Massenstrom [kg/h] | 12612,2 | 6714,1 | 5898,1 |
| Massenanteile [kg/kg] | | | |
| Dimethylether | 0,000394 | 0,000888 | |
| Isobuan | 0,019822 | 0,037235 | |
| Isobuten | 0,006077 | 0,000169 | 0,000001 |
| 1-Buten | 0,269581 | 0,506313 | 0,000099 |
| 1,3-Butadien | 0,001189 | 0,002233 | 0,000001 |
| n-Butan | 0,063431 | 0,119125 | 0,000032 |
| 2-Butene | 0,160274 | 0,299307 | 0,000844 |
| C5-Kohlenwasserstoffe | 0,000336 | | 0,000719 |
| MTBE | 0,447633 | | 0,976586 |
| 2-Methoxybutan | 0,001678 | | 0,005414 |
| Methanol | 0,025947 | 0,034285 | 0,008509 |
| Tert.-Butanol | 0,002298 | | 0,005518 |
| Wasser | 0,000278 | 0,000445 | 0,000007 |
| Diisobuten | 0,001061 | | 0,002269 |

Für die MTBE-Destillation wird die Variante [b3] gemäß Figur 5 angenommen. Demnach wird der Reaktoraustrag (IV) der Kolonne T-21 zugeführt. Die Kolonne ist als Reaktivdestillation ausgeführt. Die Kolonne hat, inklusive der Katalysatorzone, 75 theoretische Stufen, die Katalysatorzone ist auf Stufe 15 bis Stufe 50 angeordnet und der Zulauf erfolgt oberhalb Stufe 60, gezählt von oben. Das Katalysatorvolumen beträgt 0,2 m3 pro Stufe, wobei eine KataMax®-Packung (Koch-Glitsch), gefüllt mit Amberlyst® 15 (Rohm & Haas), angenommen wird. Die Kolonne wird bei einem Rücklaufverhältnis von 0,9 und bei einem Druck von 0,6 MPa(abs) betrieben. Die Kopftemperatur beträgt 51,4 °C, die Sumpftemperatur beträgt 120,9 °C. Der Umsatz an Isobuten in der Reaktivdestillationskolonne beträgt unter diesen Randbedingungen 98 %.

Das Sumpfprodukt (XVI) besteht überwiegend aus MTBE (ca. 98 Massen-%) und genügt marktgängigen Spezifikationen für Kraftstoff MTBE. Insbesondere liegt der Gehalt an TBA bei kleiner 1 Massen-%, siehe Tabelle 6. Das Destillat (X) besteht überwiegend aus C4-Kohlenwasserstoffen sowie aus Methanol, in der MTBE-Synthese gebildetem DME sowie Wasser. Durch eine Extraktion mit Wasser kann das Methanol entfernt werden, wie in Figur 5 dargestellt. Die aus der Extraktion erthaltenen, methanolfreien C4-Kohlenwasserstoffe (XIII) können nach bekannten Verfahren zu hochreinem 1-Buten aufgearbeitet werden. In diesem Zusammenhang ist wichtig, dass der Isobutengehalt, bezogen auf den 1-Butengehalt, in Strom (X) kleiner als 350 ppm beträgt. Damit kann, nach Abtrennung bzw. Umsetzung von Buatdien in einer selektiven Hydrierung, durch ein rein destillatives Verfahren ein hochreines 1-Buten gewonnnen werden, dass einer marktgängigen Isobutenspezifikation genügt (z. B. < 2000 pppm Isobuten im 1-Buten).

**Tabelle 7: Zusammensetzung des Destillats (L) und des Sumpfproduktes (XVIII) der Kolonne T-22 sowie des Sumpfproduktes (XV) der Kolonne T-23 für Beispiel 2.**

| | Destillat T-22 (L) | Sumpfprod. T-22 (XVIII) | Destillat T-23 (V) | Sumpfprod. T-23 (XV) |
|---|---|---|---|---|
| Massenstrom [kg/h] | 6,7 | 5891,4 | 5747,4 | 143,9 |
| Massenanteile [kg/kg] | | | | |
| Dimethylether | | | | |
| Isobuan | 0,000158 | | | |
| Isobuten | 0,001252 | | | |
| 1-Buten | 0,086129 | 0,000001 | 0,000001 | |
| 1,3-Butadien | 0,000712 | | | |
| n-Butan | 0,028309 | | | |
| 2-Butene | 0,677852 | 0,000071 | 0,000073 | |
| C5-Kohlenwasserstoffe | 0,036666 | 0,000678 | 0,000695 | |
| MTBE | 0,100000 | 0,977588 | 0,982811 | 0,769032 |
| 2-Methoxybutan | 0,000020 | 0,005420 | 0,002100 | 0,137986 |
| Methanol | 0,067130 | 0,008442 | 0,008653 | |
| Tert.-Butanol | | 0,005525 | 0,005662 | 0,000023 |
| Wasser | 0,001771 | 0,000005 | 0,000005 | |
| Diisobuten | | 0,002271 | | 0,092959 |

Der MTBE-Strom (XVI) enthält in Summe noch ca. 1700 ppm C4- und C5-Kohlenwasserstoffe. Aus diesem Strom werden in der Kolonne T-22 diese C4- und C5 Kohlenwasserstoffe bis auf einen Restgehalt von 750 Massen-ppm abgetrennt. Die Kolonne hat 52 theoretische Stufen und wird bei einem Rücklaufverhältnis von 212 und bei einem Druck von 4,5 MPa(abs) betrieben. Der Zulauf (XVI) erfolgt oberhalb von Stufe 22, gezählt von oben. Die Kopftemperatur beträgt 48,1 °C, die Sumpftemperatur beträgt 107,3 °C. Das Destillat dieser Kolonne (L) hat einen Restgehalt von 10 Massen-% MTBE. Durch Erhöhung des Rücklaufverhältnisses und/oder der Trennstufenzahl ließe sich der MTBE-Gehalt weiter reduzieren. Tabelle 7 zeigt die Zusammensetzung des Destillatstroms (L) und des Sumpfstroms (XVIII) der Kolonne T-22.

Die weitestgehend von Leichtsiedern befreite MTBE-Strom (XVIII) wird der Kolonne T-23 zugeführt, in der vornehmlich Diisobuten und 2-Methoxybutan über Sumpf (XV) abgetrennt werden. Die Kolonne hat 95 theoretische Stufen und wird bei einem Rücklaufverhältnis von 5,9 und bei einem Druck von 0,95 MPa(abs) betrieben. Die Zugabe von Strom (XVIII) erfolgt oberhalb Stufe 28, von oben gezählt. Die Kopftemperatur beträgt 144,1 °C, die Sumpftemperatur beträgt 149,16 °C. Als Kopfprodukt (V) wird eine gasförmige Fraktion erhalten, die über 98 Massen-% MTBE enthält. Der Gehalt an 2-Methoxybutan im Destillat wurde auf 2100 Massen-ppm eingestellt (s. Tabelle 7). Durch Erhöhung des Rücklaufverhältnisses und/oder der Trennleistung ließe sich der Gehalt an MTBE im Sumpfprodukt (XV) reduzieren. Durch die Fahrweise der Kolonne bei erhöhtem Druck wird nahezu das gesamte im Zulauf vorhandene TBA ins Kopfprodukt (V) destilliert.

Die MTBE-Fraktion (V) wird, nach weiterer Aufheizung auf Reaktionstemperatur, gemäß Figur 1 dem Spaltungsreaktor in Verfahrensschritt [c] zugeführt. Der Spaltungsreaktor wird mit einem Reaktorvolumen von 5,5 m3 modelliert, wobei eine Füllung mit einem Katalysator angenommen wird, der formal aus Magnesiumoxid, Aluminiumoxid und Siliziumoxid besteht und dessen Herstellung im Patent DE 102006040432.7 beschrieben ist.

Der Reaktor wird bei 298 °C und 0,75 MPa(abs) gefahren. Bei diesen Reaktionsbedingungen ergibt sich ein MTBE-Umsatz von ca. 94 %, der Umsatz von 2-Methoxybutan beträgt ca. 17 %. Aufgrund der Begrenzung des Anteils an 2-Methoxybutan auf 2100 Massen-ppm im Reaktorzulauf ist aber trotz der Spaltung von 2-Methoxybutan zu 2-Buten eine marktübliche Spezifikation für lineare Butene im Isobutenprodukt nicht gefährdet. Der Reaktoraustrag enthält durch DME-Bildung und TBA-Spaltung entstandenes Wasser, ca. 6000 ppm. Die Zusammensetzung des Reaktoraustrags (IV) zeigt Tabelle 8.

**Tabelle 8: Zusammensetzung des Produkts (VI) des Spaltreaktors, des Destillatstroms (XVII) und des Sumpfstroms (VII) der Kolonne T-41 sowie des Sumpfprodukts (XLI) der Kolonne T-51 für Beispiel 2.**

| | Produkt Spaltreaktor (VI) | Destillat T-41 (XVII) | Sumpfprod. T-41 (VII) | Sumpfprod. T-51 (XLI) |
|---|---|---|---|---|
| Massenstrom [kg/h] | 5747,4 | 3610,3 | 2137,1 | 27,7 |
| Massenanteile [kg/kg] | | | | |
| Dimethylether | 0,011201 | 0,017817 | 0,000025 | |
| Isobuan | | | | |
| Isobuten | 0,594182 | 0,945788 | 0,000200 | |
| 1-Buten | 0,000001 | 0,000001 | | |
| 1,3-Butadien | | | | |
| n-Butan | | | | |
| 2-Butene | 0,000301 | 0,000479 | 0,000001 | |
| C5-Kohlenwasserstoffe | 0,000695 | 0,000347 | 0,001283 | |
| MTBE | 0,054055 | | 0,145371 | |
| 2-Methoxybutan | 0,001741 | | 0,004682 | |
| Methanol | 0,330802 | 0,034738 | 0,830956 | 0,000005 |
| Tert.-Butanol | 0,000493 | | 0,001325 | |
| Wasser | 0,005642 | 0,000829 | 0,013773 | 0,999995 |
| Diisobuten | 0,000887 | | 0,002385 | |

Für die Aufreinigung der Spaltprodukte wird die Variante [d1] gemäß Figur 8 angenommen. Demnach wird der Reaktoraustrag (IV) teilweise kondensiert und zweiphasig der Kolonne T-41 zugeführt. Die Kolonne hat 42 theoretische Stufen und wird bei einem Rücklaufverhältnis von 0,3 und bei einem Druck von 0,65 MPa(abs) betrieben. Die Zugabe des Zulaufstromes (VI) erfolgt oberhalb Stufe 28, gezählt von oben. Die Kopftemperatur beträgt 50,8 °C, die Sumpftemperatur beträgt 117,0 °C. Das Sumpfprodukt besteht überwiegend aus nicht umgesetztem MTBE (ca. 15 Massen-%) und Methanol (ca. 83 Massen-%) sowie dem überwiegenden Teil der im Zulaufstrom enthaltenen Wassermenge (ca. 1,4 Massen-%), siehe Tabelle 8.

Das Kopfprodukt (XVII) ist Isobuten mit einer Reinheit von größer 94 Massen-% Isobuten. Die in einer typischen Isobutenspezifikation geforderten Grenzen für lineare Butene (< 1000 Massen-ppm) und C5-Kohlenwasserstoffe (< 1000 Massen-ppm) werden sicher eingehalten, siehe auch Tabelle 3. Wie in Figur 12 dargestellt, kann durch eine Extraktion mit Wasser bei Bedarf das Methanol entfernt werden. Das Restwasser und der Dimethylether können durch eine anschließende Destillation abgetrennt und das Isobuten (XLIX) auf eine Reinheit größer 99,9 Massen-% aufkonzentriert werden.

Für die Trocknung von Strom (VII) wird die Variante [e1] gemäß Figur 10 angenommen. Demnach wird Sumpfstrom (VII) der Kolonne T-41 der Kolonne T-51 zugeführt. Die Kolonne hat 32 theoretische Stufen und wird bei einem Rücklaufverhältnis von 0.7 und bei einem Druck von 0,11 MPa(abs) betrieben. Die Zugabe des Zulaufstromes (VII) erfolgt oberhalb Stufe 14, gezählt von oben. Die Kopftemperatur beträgt 61,7 °C, die Sumpftemperatur beträgt 102,3 °C.

Das Wasser wird aus dem Destillatstrom bis auf einen Restgehalt von 800 ppm abgetrennt (siehe Strom (VIII) in Tabelle 5) und das Destillat wird in Verfahrensschritt a) zurückgeführt. Der Sumpfstrom der Kolonne besteht aus praktisch reinem Wasser mit einem Restgehalt von 5 ppm Methanol.

### Beispiel 3: (nicht erfindungsgemäß)

Das Beispiel 3 dient als Vergleichsbeispiel und beinhaltet nicht das erfindungsgemäße Verfahren. Demnach wird ein Verfahren nach Figur 1 betrachtet, wobei allerdings Verfahrschritt [e], d.h. die Trocknung des Rückführstromes (VII) und die entsprechende Wasser-Abtrennung, entfällt. Die Umsetzung des Isobutens erfolgt analog zu Beispiel 2 nach einer Variante [a1] gemäß Figur 2, die MTBE-Destillation nach einer Variante [b3] gemäß Figur 5 und die Aufreinigung der Spaltprodukte nach einer Variante [d1] gemäß Figur 8. Der Strom (VII) aus Verfahrensschritt [d] wird in Beispiel 3 in Verfahrensschritt [a] zurückgeführt.

Als Zulauf zur Anlage wird analog zu Beispiel 2 ein Strom (II) von 10.000 kg/h angenommen. Die Zusammensetzungen des Stromes (II), des zugeführten Frischmethanols sowie des aus Verfahrenschritt [d] kommenden des Rückführstroms (VII) sind in Tabelle 9 dargestellt. Die Zusammensetzungen von Strom (II) und Strom (IX) sind gegenüber Beispiel 2 unverändert, siehe Tabelle 5. Die Frischmethanolmenge wurde wieder so eingestellt, dass sich ein molares Verhältnis von Methanol zu Isobuten im Zulauf zum ersten Reaktor von 1,14 ergibt.

**Tabelle 9: Zusammensetzung des Eintrittsstroms (II), des Frischmethanols (IX) und des Rückführstroms (VII) für Beispiel 3.**

| | Raffinat I (II) | Frisch-methanol (IX) | Rückführstrom (VII) |
|---|---|---|---|
| Massenstrom [kg/h] | 10000,0 | 518,1 | 2109,1 |
| Massenanteile [kg/kg] | | | |
| Dimethylether | | | 0,000056 |
| Isobuan | 0,025000 | | |
| Isobuten | 0,350000 | | 0,000200 |
| 1-Buten | 0,340000 | | |
| 1,3-Butadien | 0,001500 | | |
| n-Butan | 0,080000 | | |
| 2-Butene | 0,202850 | | 0,000001 |
| C5-Kohlenwasserstoffe | 0,000150 | | 0,001097 |
| MTBE | | | 0,148802 |
| 2-Methoxybutan | | | 0,004200 |
| Methanol | | 0,997500 | 0,832828 |
| Tert.-Butanol | | | 0,000008 |
| Wasser | 0,000500 | 0,000250 | 0,010962 |
| Diisobuten | | | 0,001845 |

Der C4-Kohlenwasserstoffstrom (II), das Frischmethanol (IX) und der methanolhaltige Rückführungsstrom (VII) werden vermischt und in Variante [a1] gemäß Figur 2 den Reaktoren R-11, R-12 und R-13 zugeführt. Die Anordnung der Reaktoren, die Reaktorgrößen sowie die Eintrittstemperaturen sind gegenüber Beispiel 2 unverändert.

Der Rückführstrom (VII) enthält gegenüber Beispiel 2 (dort Strom (VIII)) mit ca. 1,1 Massen-% wesentlich mehr Wasser. Wie aus Beispiel 1 ersichtlich, sinkt bei höheren Wasserkonzentrationen unter sonst gleichen Randbedingungen der Isobuten-Umsatz zu MTBE. Folglich ergibt sich in Beispiel 3 ein Umsatz von Isobuten über alle drei Reaktoren von nur ca. 68,1 % (97,8 % in Beispiel 2). Die entsprechende Zusammensetzung des Reaktoraustrags (IV) ist in Tabelle 10 aufgeführt (Zulauf zur Kolonne T-21). U. a. ist auch der TBA-Gehalt im Reaktoraustrags (IV) in Beispiel 3 mit ca. 8000 ppm deutlich höher (ca. 2200 ppm in Beispiel 2).

**Tabelle 10: Zusammensetzung des Zulaufstroms (IV), des Destillats und des Sumpfproduktes (XVI) der Kolonne T-21 für Beispiel 3.**

| | Zulauf T-21 (IV) | Destillat T-21 (X) | Sumpfprod. T-21 (XVI) |
|---|---|---|---|
| Massenstrom [kg/h] | 12627,2 | 7371,0 | 5256,2 |
| Massenanteile [kg/kg] | | | |
| Dimethylether | 0,000659 | 0,001507 | |
| Isobuan | 0,019799 | 0,033916 | |
| Isobuten | 0,088515 | 0,080591 | 0,000016 |
| 1-Buten | 0,269260 | 0,461209 | 0,000077 |
| 1,3-Butadien | 0,001188 | 0,002034 | 0,000001 |
| n-Butan | 0,063355 | 0,108517 | 0,000023 |
| 2-Butene | 0,160304 | 0,273468 | 0,000777 |
| C5-Kohlenwasserstoffe | 0,000302 | | 0,000726 |
| MTBE | 0,311137 | | 0,902868 |
| 2-Methoxybutan | 0,001238 | | 0,004278 |
| Methanol | 0,074873 | 0,038018 | 0,068855 |
| Tert.-Butanol | 0,008065 | | 0,020294 |
| Wasser | 0,000624 | 0,000739 | 0,000447 |
| Diisobuten | 0,000683 | | 0,001640 |

Für die MTBE-Destillation wird wieder die Variante [b3] gemäß Figur 5 angenommen, wobei die Kolonne T-21 wie in Beispiel 2 als Reaktivdestillation ausgeführt ist. Theoretische Stufenzahl, Katalysatorvolumen, Rücklaufverhältnis und Kolonnendruck bleiben gegenüber Beispiel 2 unverändert. Die Kopftemperatur beträgt 51,0 °C, die Sumpftemperatur beträgt 110,7 °C. Der Umsatz an Isobuten in der Reaktivdestillationskolonne beträgt unter diesen Randbedingungen ca. 47 %.

Das Sumpfprodukt (XVI) besteht überwiegend aus MTBE (ca. 90 Massen-%) Methanol (ca. 7 Massen-%) und TBA (ca. 2 Massen-%). Damit genügt Strom nicht marktgängigen Spezifikationen für Kraftstoff MTBE (typische Spezifikation TBA-Gehalt kleiner 1 Massen-% und Methanol-Gehalt kleiner 1 Massen-%). Das Destillat (X) besteht überwiegend aus C4-Kohlenwasserstoffen sowie aus Methanol, in der MTBE-Synthese gebildetem DME sowie Wasser. Durch eine Extraktion mit Wasser kann das Methanol entfernt werden, wie in Figur 5 dargestellt. Aufgrund der Verschlechterung des Isobuten-Umsatzes infolge des erhöhten Wassergehaltes in der MTBE-Synthese liegt Isobutengehalt, bezogen auf den 1-Butengehalt, in Strom (X) bei ca. 17 %. Damit können die methanolfreien C4-Kohlenwasserstoffe (XIII) nach der Extraktion nicht direkt nach bekannten Verfahren zu hochreinem 1-Buten aufgearbeitet werden. Nach Abtrennung bzw. Umsetzung von Buatdien in einer selektiven Hydrierung kann hochreines 1-Buten (typische Spezifikation kleiner 2000 ppm Isobuten im 1-Buten) nicht durch ein rein destillatives Verfahren gewonnnen werden. Auch eine Umsetzung des im 1-Buten vorhandenen Isobutens in einer zweiten MTBE-Stufe (R-22 in Figur 7) ist nicht möglich, da der Isobutengehalt aufgrund der Limitierung durch das Gleichgewicht deutlich zu hoch ist, um in einfachen Festbettreaktoren, ggf. auch mit Reaktivdestillation, die gewünschte Spezifikation zu erreichen.

**Tabelle 11: Zusammensetzung des Destillats (L) und des Sumpfproduktes (XVIII) der Kolonne T-22 sowie des Sumpfproduktes (XV) der Kolonne T-23 für Beispiel 3.**

| | Destillat T-22 (L) | Sumpfprod. T-22 (XVIII) | Destillat T-23 (V) | Sumpfprod. T-23 (XV) |
|---|---|---|---|---|
| Massenstrom [kg/h] | 5,5 | 5250,7 | 5028,8 | 221,9 |
| Massenanteile [kg/kg] | | | | |
| Dimethylether | | | | |
| Isobuan | 0,000099 | | | |
| Isobuten | 0,015489 | | | |
| 1-Buten | 0,073655 | 0,000001 | 0,000001 | |
| 1,3-Butadien | 0,000660 | | | |
| n-Butan | 0,021950 | | | |
| 2-Butene | 0,673179 | 0,000076 | 0,000079 | |
| C5-Kohlenwasserstoffe | 0,050616 | 0,000674 | 0,000703 | |
| MTBE | 0,100000 | 0,903705 | 0,924709 | 0,427689 |
| 2-Methoxybutan | 0,000017 | 0,004282 | 0,002100 | 0,053740 |
| Methanol | 0,047796 | 0,068876 | 0,071916 | |
| Tert.-Butanol | | 0,020315 | 0,000043 | 0,479732 |
| Wasser | 0,016538 | 0,000430 | 0,000449 | |
| Diisobuten | | 0,001641 | | 0,038839 |

Der MTBE-Strom (XVI) enthält in Summe noch ca. 1600 ppm C4- und C5-Kohlenwasserstoffe. Aus diesem Strom werden in der Kolonne T-22 diese C4- und C5 Kohlenwasserstoffe bis auf einen Restgehalt von 750 Massen-ppm abgetrennt. Die Kolonne wird bei einem Rücklaufverhältnis von 192 betrieben, theoretische Stufenzahl und Kolonnendruck sind gegenüber Beispiel 2 unverändert. Die Kopftemperatur beträgt 48,3 °C, die Sumpftemperatur beträgt 100,8 °C. Tabelle 11 zeigt die Zusammensetzung des Destillatstroms (L) und des Sumpfstroms (XVIII) der Kolonne T-22.

Die weitestgehend von Leichtsiedern befreite MTBE-Strom (XVIII) wird der Kolonne T-23 zugeführt. In Beispiel 3 fehlt der erfindungsgemäße Schritt der Wasser-Abtrennung gemäß Verfahrensschritt e). Damit wird das das durch TBA-Spaltung und DME-Bildung in der Etherspaltung gebildete Wasser komplett zurück in die Synthese gefahren, dort zu mehr als 50 % wieder zu TBA umgewandelt und so, in TBA gebunden, zurück in die Spaltung gefahren. Ohne TBA-Aussschleusung würde sich daher TBA bzw. Wasser im Verfahren immer weiter anreichern, ein stationärer Betrieb wäre nicht möglich. Aus diesem Grund wird in Beispiel 3 in der Kolonne T-23 neben Diisobuten und 2-Methoxybutan über Sumpf (XV) auch TBA abgetrennt. Dazu wird der Druck in der Kolonne gegenüber Beispiel 2 0,2 MPa(abs) abgesenkt, die theoretische Stufenzahl bleibt unverändert. Die Kolonne wird bei einem Rücklaufverhältnis von 2,6 betrieben. Die Kopftemperatur beträgt 73,2° C, die Sumpftemperatur beträgt 87,6 °C. Aufgrund des Verschwindens des Druckazeotrops zwischen MTBE und TBA bei niedrigen Drücken wird TBA in dieser Fahrweise über Sumpf ausgeschleust. Der MTBE-Gehalt im Sumpf wurde so eingestellt, dass, wie in Beispiel 2, 2 % des im Zulaufstroms (XVIII) enthaltenen MTBEs über Sumpf gefahren werden. Als Kopfprodukt (V) wird eine flüssige Fraktion erhalten, die über 92 Massen-% MTBE und 8 Massen-% Methanol enthält. Der Gehalt an 2-Methoxybutan im Destillat wurde, wie in Beispiel 2 auf 2100 Massen-ppm eingestellt (s. Tabelle 11).

Im Gegensatz zu Beispiel 2 kann aufgrund des niedrigen Druckes der Kolonne T-23 die MTBE-Fraktion (V) nicht gasförmig, ohne Verdichtung, in der Spaltungsreaktor in Verfahrensschritt [c] geführt werden. Daher wird in Beispiel 3 die MTBE-Fraktion (V) aus der Kolonne T-23 flüssig abgezogen, auf eine Druck größer Reaktionsdruck verdichtet, vollständig verdampf und schließlich nach weiterer Aufheizung auf Reaktionstemperatur dem Spaltungsreaktor zugeführt. Das Volumen und der Druck des Spaltungsreaktor sind gegenüber Beispiel 2 unverändert. Der Reaktor wird bei 293 °C gefahren. Bei diesen Reaktionsbedingungen ergibt sich ein MTBE-Umsatz von ca. 93 %, der Umsatz von 2-Methoxybutan beträgt ca. 16 %. Die Zusammensetzung des Reaktoraustrags (IV) zeigt Tabelle 12.

**Tabelle 12: Zusammensetzung des Produkts (VI) des Spaltreaktors und des Destillatstroms (XVII) der Kolonne T-41 für Beispiel 3.**

| | Produkt Spaltreaktor (VI) | Destillat T-41 (XVII) |
|---|---|---|
| Massenstrom [kg/h] | 5028,8 | 2919,7 |
| Massenanteile [kg/kg] | | |
| Dimethylether | 0,011522 | 0,019804 |
| Isobutan | | |
| Isobuten | 0,548113 | 0,943912 |
| 1-Buten | 0,000001 | 0,000001 |
| 1,3-Butadien | | |
| n-Butan | | |
| 2-Butene | 0,000294 | 0,000506 |
| C5-Kohlenwasserstoffe | 0,000703 | 0,000419 |
| MTBE | 0,062409 | |
| 2-Methoxybutan | 0,001762 | |
| Methanol | 0,369455 | 0,034726 |
| Tert.-Butanol | 0,000003 | |
| Wasser | 0,004964 | 0,000631 |
| Diisobuten | 0,000774 | |

Für die Aufreinigung der Spaltprodukte wird, wie in Beispiel 2, die Variante [d1] gemäß Figur 8 angenommen. Theoretische Stufenzahl, Kolonnendruck und Rücklaufverhältnis der Kolonne T-41 sind gegenüber Beispiel 2 unverändert. Die Kopftemperatur beträgt 50,8 °C, die Sumpftemperatur beträgt 116,9 °C. Das Sumpfprodukt besteht (VII) überwiegend aus nicht umgesetztem MTBE (ca. 15 Massen-%) und Methanol (ca. 83 Massen-%) sowie der überwiegenden Teil der im Zulaufstrom enthaltenen Wassermenge (ca. 1,1 Massen-%), siehe Tabelle 9. Strom (VII) wird in Beispiel 3 direkt in Verschritt a) zurückgeführt.

Durch die Gegenüberstellung von Beispiel 2 (erfindungsgemäß) und Beispiel 3 (nicht erfindungsgemäß) konnten sehr anschaulich die Vorteile des erfindungsgemäßen Verfahrens aufgezeigt werden. Durch den fehlenden Verfahrensschritt e) geht in Beispiel 3 der Isobuten-Umsatz aufgrund des erhöhten Wasseranteils in der MTBE-Synthese und der damit verbundenen Reaktionshemmung deutlich zurück, soweit, dass unter gleichen Randbedingungen weder spezifikationsgerechtes 1-Buten noch spezifikationsgerechtes Kraftstoff-MTBE produziert werden können. Weiterhin wird durch den fehlenden Verfahrenssschritt e) eine Aussschleusung von TBA in Verfahrensschritt b) notwendig. Dies führt zum einen zu einem erhöhten Isobuten-Verlust durch das im TBA gebundene Isobuten, zum anderen zu einem erhöhten Energiebedarf, da bei niedrigem Druck an der Kolonne T-23 ein gasförmiger Abzug und eine direkte Zuführung in der Spaltungsreaktor nicht mehr möglich ist.

Führt man in der Etherspaltung Wasserdampf zu, um die Reaktion positiv zu beeinflussen, oder dosiert man in die Verfahrensschritte nach der Etherspaltung Lauge zu, um Korrosion zu vermeiden, verstärken sich die Nachteile eines Verfahrens ohne die erfindungsgemäße Wasser-Abtrennung gemäß Verfahrensschritt e) noch weiter.

## Patentansprüche

1. Verfahren zur Herstellung von Isobuten durch Spaltung von MTBE, wobei folgende Schritte durchlaufen werden:
a) MTBE-Synthese; Umsetzung von Isobuten-haltigen Kohlenwasserstoffgemischen (II), mit Methanol (III), enthalten in einem oder mehreren Methanol-haltigen Strömen (VIII, IX), an sauren Ionentauschern unter Erhalt eines Stroms (IV), enthaltend MTBE und TBA,
b) MTBE-Abtrennung; destillative Abtrennung eines Stroms (V), enthaltend MTBE und TBA, aus Strom (IV),
c) MTBE-Spaltung; Spaltung des Stroms (V) an einem heterogenen Katalysator in der Gasphase unter Erhalt eines Stroms (VI), enthaltend mindestens Isobuten, Methanol, MTBE und Wasser und gegebenenfalls TBA,
d) Isobuten-Abtrennung; destillative Trennung des Stroms (VI) unter Erhalt eines Stroms (VII), enthaltend jeweils mehr als 50 Massen-% der in Strom (VI) enthaltenen Methanol-, TBA- und Wasser-Mengen, und eines Stroms (XVII), enthaltend Isobuten,
e) Wasser-Abtrennung; destillative Abtrennung von Wasser aus dem Strom (VII) auf unter 1 Massen-% Anteil unter Erhalt eines Stroms (VIII),
f) Rückführung; vollständige oder teilweise Rückführung des methanolhaltigen Stroms (VIII) in die MTBE-Synthese.

2. Verfahren nach Anspruch 1,
**dadurch gekennzeichnet,**
**dass** die Wasser-Abtrennung aus Strom (VII) in zwei Kolonnen durchgeführt wird, wobei die erste bei niedrigerem Druck betrieben wird und ihr Sumpfprodukt in einer zweiten Kolonne, die bei höherem Druck betrieben wird, weiter aufgereinigt wird.

3. Verfahren nach den Ansprüchen 1 und 2,
**dadurch gekennzeichnet,**
**dass** die erste Kolonne zumindest teilweise über die Brüden der zweiten Kolonne beheizt wird.

4. Verfahren nach einem der Ansprüche 1 bis 3,
**dadurch gekennzeichnet,**
**dass** in der zweiten Kolonne zusätzliche Wasser-Methanol-Mischungen aufdestilliert werden.

5. Verfahren nach zumindest einem der Ansprüche 1 bis 4,
**dadurch gekennzeichnet,**
**dass** dem Strom (V) Wasser oder Wasserdampf zugegeben wird

6. Verfahren nach zumindest einem der Ansprüche 1 bis 5,
**dadurch gekennzeichnet,**
**dass** bei der Isobuten-Abtrennung und/ oder der Wasser-Abtrennung ein Laugestrom zugegeben wird.

7. Verfahren nach Anspruch 6,
**dadurch gekennzeichnet,**
**dass** für die Laugenzugabe wässrige Lösungen von Alkalimetall- oder Erdalkalimetallhydroxiden eingesetzt werden.

8. Verfahren nach einem der Ansprüche 1 bis 6,
**dadurch gekennzeichnet,**
**dass** die Lauge bei der Wasser-Abtrennung zusammen mit dem abgetrennten Wasser entfernt wird.

9. Verfahren nach einem der Ansprüche 1 bis 8,
**dadurch gekennzeichnet,**
**dass** bei der Isobuten-Abtrennung eine oder zwei Destillationskolonnen eingesetzt werden, und dass die Laugenzugabe in den Zulauf der Kolonne bzw. in den Zulauf der ersten Kolonne erfolgt.

10. Verfahren nach Anspruch 6 und zumindest einem der Ansprüche 1 bis 9, **dadurch gekennzeichnet,**
**dass** bei der Isobuten-Abtrennung eine oder zwei Destillationskolonnen eingesetzt werden, und dass die Laugenzugabe in die Kolonne bzw. in die erste Kolonne unterhalb der Zugabe des Zulaufs erfolgt.

11. Verfahren nach Anspruch 6 und zumindest einem der Ansprüche 1 bis 10,
**dadurch gekennzeichnet,**
**dass** bei der Wasser-Abtrennung eine oder zwei Destillationskolonnen eingesetzt werden, und dass die Laugedosierung in den Zulauf der Kolonne bzw. in den Zulauf der ersten Kolonne erfolgt.

12. Verfahren nach Anspruch 6 und zumindest einem der Ansprüche 1 bis 11,
**dadurch gekennzeichnet,**
**dass** bei der Wasser-Abtrennung eine oder zwei Destillationskolonnen eingesetzt werden, und dass die Laugenzugabe in die Kolonne bzw. in die erste Kolonne unterhalb der Zugabe des Zulaufs erfolgt.

13. Verfahren nach zumindest einem der Ansprüche 1 bis 12,
**dadurch gekennzeichnet,**
**dass** die Kolonne, in der der Reaktoraustrag überführt wird, bei einem niedrigeren Druck als der Reaktor betrieben wird.

14. Verfahren nach zumindest einem der Ansprüche 1 bis 13,
**dadurch gekennzeichnet,**
**dass** die Wärme aus der Abkühlung und teilweisen Kondensation des Reaktoraustrags zur Wärmeintegration innerhalb des Prozesses genutzt wird.

## Claims

1. Process for preparing isobutene by dissociation of MTBE, which comprises the following steps:
a) MTBE synthesis; reaction of isobutene-containing hydrocarbon mixtures (II), with methanol (III), present in one or more methanol-containing streams (VIII, IX), over acidic ion exchangers to give a stream (IV), containing MTBE and TBA,
b) Isolation of MTBE; separation of a stream (V), containing MTBE and TBA, from stream (IV) by distillation,
c) MTBE dissociation; dissociation of stream (V) in the gas phase over a heterogeneous catalyst to give a stream (VI) containing at least isobutene, methanol, MTBE and water and possibly TBA,
d) Isolation of isobutene; separation of the stream (VI) by distillation to give a stream (VII), containing in each case more than 50% by mass of the amounts of methanol, TBA and water present in stream (VI) and a stream (XVII) containing isobutene,
e) Removal of water; separation of water from stream (VII) to below 1% by mass by distillation to give a stream (VIII),
f) Recirculation; total or partial recirculation of the methanol-containing stream (VIII) to the MTBE synthesis.

2. Process according to Claim 1,
**characterized in that**
the removal of water from stream (VII) is carried out in two columns, with the first being operated at a lower pressure and the bottom product therefrom being purified further in a second column operated at a higher pressure.

3. Process according to Claim 1 or 2,
**characterized in that**
the first column is at least partly heated by means of the vapours from the second column.

4. Process according to any of Claims 1 to 3,
**characterized in that**
additional water/methanol mixtures are distilled in the second column.

5. Process according to at least one of Claims 1 to 4,
**characterized in that**
water or water vapour is added to the stream (V).

6. Process according to at least one of Claims 1 to 5,
**characterized in that**
a stream of alkali is introduced in the isolation of isobutene and/or the removal of water.

7. Process according to Claim 6,
**characterized in that**
aqueous solutions of alkali metal hydroxides or alkaline earth metal hydroxides are used for the addition of alkali.

8. Process according to any of Claims 1 to 6,
**characterized in that**
the alkali is removed in the removal of water together with the water which is separated off.

9. Process according to any of Claims 1 to 8,
**characterized in that**
one or two distillation columns are used in the isolation of isobutene and **in that** the addition of alkali is effected by introduction into the feed to the column or into the feed to the first column.

10. Process according to Claim 6 and at least one of Claims 1 to 9,
**characterized in that**
one or two distillation columns are used in the isolation of isobutene and **in that** the addition of alkali is effected by introduction into the column or into the first column below the point of introduction of the feed.

11. Process according to Claim 6 and at least one of Claims 1 to 10,
**characterized in that**
one or two distillation columns are used in the removal of water and **in that** the introduction of alkali is effected into the feed to the column or into the feed to the first column.

12. Process according to Claim 6 and at least one of Claims 1 to 11,
**characterized in that**
one of two distillation columns are used in the removal of water and **in that** the addition of alkali is effected by introduction into the column or into the first column below the point of introduction of the feed.

13. Process according to at least one of Claims 1 to 12,
**characterized in that**
the column, into which the reactor output is transferred is operated at a lower pressure than the reactor.

14. Process according to at least one of Claims 1 to 13,
**characterized in that**
the heat from the cooling and partial condensation of the reactor output is utilized for heat integration within the process.

## Revendications

1. Procédé pour la préparation d'isobutène par dissociation de MTBE, les étapes suivantes étant parcourues :
a) synthèse de MTBE ; transformation de mélanges d'hydrocarbures (II) contenant de l'isobutène avec du méthanol (III), contenu dans un ou plusieurs flux (VIII, IX) contenant du méthanol, sur des échangeurs d'ions acides avec obtention d'un flux (IV) contenant du MTBE et TBA,
b) séparation de MTBE ; séparation par distillation d'un flux (V), contenant du MTBE et du TBA, du flux (IV),
c) dissociation de MTBE ; dissociation du flux (V) sur un catalyseur hétérogène en phase gazeuse avec obtention d'un flux (VI), contenant au moins de l'isobutène, du méthanol, du MTBE et de l'eau et le cas échéant du TBA,
d) séparation de l'isobutène ; séparation par distillation du flux (VI) avec obtention d'un flux (VII), contenant à chaque fois plus de 50% en masse des quantités de méthanol, de TBA et d'eau contenues dans le flux (VI), et d'un flux (XVII), contenant de l'isobutène,
e) séparation de l'eau ; séparation par distillation de l'eau du flux (VII) à une proportion inférieure à 1% en masse avec obtention d'un flux (VIII),
f) recyclage ; recyclage total ou partiel du flux contenant du méthanol (VIII) dans la synthèse de MTBE.

2. Procédé selon la revendication 1, **caractérisé en ce que** la séparation de l'eau du flux (VII) est réalisée en deux colonnes, la première colonne étant exploitée à une pression inférieure et son produit de fond étant purifié davantage dans une deuxième colonne, qui est exploitée à une température supérieure.

3. Procédé selon les revendications 1 et 2, **caractérisé en ce que** la première colonne est chauffée au moins partiellement via les vapeurs de la deuxième colonne.

4. Procédé selon l'une quelconque des revendications 1 à 3, **caractérisé en ce que** des mélanges supplémentaires d'eau-méthanol sont distillés dans la deuxième colonne.

5. Procédé selon au moins l'une quelconque des revendications 1 à 4, **caractérisé en ce que** de l'eau ou de la vapeur d'eau est ajoutée au flux (V).

6. Procédé selon au moins l'une quelconque des revendications 1 à 5, **caractérisé en ce qu'**un flux de lessive est ajouté à la séparation de l'isobutène et/ou à la séparation de l'eau.

7. Procédé selon la revendication 6, **caractérisé en ce qu'**on utilise, pour l'addition de lessive, des solutions aqueuses d'hydroxydes de métal alcalin ou de métal alcalino-terreux.

8. Procédé selon l'une quelconque des revendications 1 à 6, **caractérisé en ce que** la lessive est éliminée, lors de la séparation de l'eau, avec l'eau séparée.

9. Procédé selon l'une quelconque des revendications 1 à 8, **caractérisé en ce qu'**on utilise, lors de la séparation de l'isobutène, une ou deux colonnes de distillation et **en ce que** l'addition de lessive a lieu dans l'alimentation de la colonne ou dans l'alimentation de la première colonne.

10. Procédé selon la revendication 6 et selon au moins l'une quelconque des revendications 1 à 9, **caractérisé en ce qu'**on utilise, lors de la séparation de l'isobutène, une ou deux colonnes de distillation et **en ce que** l'addition de lessive a lieu dans la colonne ou dans la première colonne sous l'addition de l'alimentation.

11. Procédé selon la revendication 6 et selon au moins l'une quelconque des revendications 1 à 10, **caractérisé en ce qu'**on utilise, lors de la séparation de l'eau, une ou deux colonnes de distillation et **en ce que** le dosage de lessive a lieu dans l'alimentation de la colonne ou dans l'alimentation de la première colonne.

12. Procédé selon la revendication 6 et selon au moins l'une quelconque des revendications 1 à 11, **caractérisé en ce qu'**on utilise, lors de la séparation de l'eau, une ou deux colonnes de distillation et **en ce que** l'addition de lessive a lieu dans la colonne ou dans la première colonne sous l'addition de l'alimentation.

13. Procédé selon au moins l'une quelconque des revendications 1 à 12, **caractérisé en ce que** la colonne, dans laquelle le produit évacué du réacteur est transféré, est exploitée à une pression inférieure à celle du réacteur.

14. Procédé selon au moins l'une quelconque des revendications 1 à 13, **caractérisé en ce que** la chaleur provenant du refroidissement et de la condensation partielle du produit évacué du réacteur est utilisée pour l'intégration de chaleur dans le procédé.
